# EUROPEAN PATENT SPECIFICATION

(11) **EP 4 117 758 B1**
(45) Date of publication and mention of the grant of the patent: **26.06.2024**
(21) Application number: 21768623.7
(22) Date of filing: 12.03.2021
(51) Int. Cl.: A61M 16/10, B01D 53/047

(54) **OXYGEN DELIVERY SYSTEM FOR THE TREATMENT OF CHRONIC OBSTRUCTIVE PULMONARY DISEASE**
SAUERSTOFFABGABESYSTEM ZUR BEHANDLUNG VON CHRONISCH OBSTRUKTIVER LUNGENKRANKHEITEN
SYSTÈME D'ADMINISTRATION D'OXYGÈNE POUR LE TRAITEMENT DES BRONCHOPNEUMOPATHIES CHRONIQUES OBSTRUCTIVES

(30) Priority: 13.03.2020 US 202062989159 P
(43) Date of publication of application: 18.01.2023
(73) Proprietor: Separation Design Group LLC, Waynesburg, PA 15370 (US); Belluscura LLC, Plano, TX 75024 (US)
(72) Inventor: GALBRAITH, Stephen Douglas, Waynesburg, PA 15370 (US); RAUKER, Robert M., Plano, TX 75024 (US)
(74) Representative: Westphal, Mussgnug & Partner Patentanwälte mbB
(86) International application number: PCT/US2021/022133
(87) International publication number: WO 2021/183903

(56) References cited:
- WO-A1-2016/174380
- WO-A1-2017/205542
- WO-A1-2018/226532
- WO-A1-2019/009771
- WO-A1-2019/221852
- FR-A1- 2 727 023
- US-A- 4 971 609
- US-A1- 2004 226 562
- US-A1- 2008 196 711
- US-A1- 2012 285 454
- US-A1- 2014 283 834
- US-A1- 2015 083 121
- US-A1- 2019 175 856
- US-A1- 2019 175 856

## Description

### BACKGROUND OF THE INVENTION

According to the World Health Organization ("WHO") over two hundred fifty million (250,000,000) people world-wide suffer from chronic obstructive pulmonary disease ("COPD") and other lung disorders. Patients with moderate to severe COPD are typically prescribed supplemental oxygen in order to raise the oxygen level in their blood. Long term administration of oxygen to patients with chronic respiratory failure has been shown to increase survival in patients with severe resting hypoxemia, which is a below-normal or low level of oxygen in the patient's blood stream.

Supplemental oxygen has been provided in the past by traditional pressurized oxygen cylinders and more recently by oxygen concentrator devices that generate ninety percent (90%) or greater purity of oxygen from ordinary or ambient air through the use of zeolites and a process called pressure swing adsorption. An example of such oxygen concentrator device is described in U.S. Patent No. 8,894,751, titled "Ultra Rapid Cycle Portable Oxygen Concentrator."

Portable supplemental oxygen allows patients to be mobile and receive the further benefit of exercise by facilitating patient exercise while carrying the portable oxygen concentrator. COPD, however, like many lung diseases is a progressive disease. Over time, patients will typically require more oxygen to maintain the appropriate blood oxygen saturation level. As more oxygen is required, the oxygen concentrators typically require larger and heavier sizes in order to generate the higher level of oxygen necessitated by the patient. Unfortunately, the higher levels of oxygen tend to be drier and cause greater irritation to the patient's respiratory tract when compared to lower levels of concentrated oxygen or no concentrated oxygen.

A recent study has shown that with certain patients, non-invasive ventilators can produce s higher fraction of inspired oxygen ("FiO₂") and increased tidal volumes compared to oxygen concentrators, both pulse dose and continuous flow. *See* McCoy, Robert, *A Bench Study to Compare Portable Therapies for Respiratory Insufficiency: Continuous Flow Oxygen (CF), Intermittent Flow Oxygen (IF), and Non-Invasive Open Ventilation (NIOV)* Valley Inspired Products. In the *McCoy* study, a non-invasive open ventilation ("NIOV") system, from Breathe Technologies, Inc. provided higher FiO₂ and higher tidal volumes than the oxygen concentrators used in the study. The NIOV system and its successor product, the LifeAir 2000, are pseudo-portable ventilator products. To be truly portable such ventilators should be capable of being non-electrically tethered for an extended period of time and be able to generate the necessary air and oxygen quantity and concentration via a portable power source such as a battery.

### Background of various lung diseases

COPD is a group of progressive lung diseases. The most common are emphysema and chronic bronchitis. Many people with COPD have both of these conditions. Emphysema slowly destroys air sacs in your lungs, which interferes with outward air flow. Bronchitis causes inflammation and narrowing of the bronchial tubes, which allows mucus to build up.

The leading cause of COPD is tobacco smoking. Long-term exposure to chemical irritants can also lead to COPD. It's a disease that usually takes a long time to develop. Diagnosis usually involves imaging tests, blood tests, and lung function tests.

There is no cure for COPD, but treatment can help ease symptoms, lower the chance of complications, and generally improve quality of life for the COPD patient. Medications, supplemental oxygen therapy, and surgery are some forms of treatment that are utilized to ease symptoms. Untreated, COPD can lead to a faster progression of the disease, heart problems, and worsening respiratory infections. It's estimated that about thirty (30) million people in the United States have COPD. As many as half are unaware that they have it.

COPD is a leading cause of hospitalizations in industrialized countries. In the United States, COPD is responsible for a large amount of emergency department visits and hospital admissions. In the year 2000, it was noted that there were over seven hundred thousand (700,000) hospital admissions and approximately one and one-half million (1,500,000) emergency department visits associated with COPD. Among people with lung cancer, between forty and seventy percent (40-70%) also have COPD.

About one hundred twenty thousand (120,000) people die from COPD each year in the United States. It's the third leading cause of death in the United States. More women than men die from COPD each year. It's projected that the number of patients diagnosed with COPD will increase by more than one hundred fifty percent (150%) from 2010 to 2030. Much of that increase can be attributed to an aging population.

The lung damage that occurs with COPD can cause hypoxia if it becomes too severe. COPD can have harmful effects on the body when it interferes with oxygen levels. If hypoxia progresses too far, it can lead to disability and death.

Oxygen passes into the blood from lung tissue through the alveoli, or air sacs. Oxygenated blood then leaves the lungs and travels around the body to other tissues. Vital organs and systems, especially the brain and heart, need enough oxygen to survive. COPD damages the air sacs in the lungs and interferes with this process. If the damage reaches a critical point, a person may develop hypoxia. Hypoxia occurs when the blood does not deliver enough oxygen to the air sacs in the lungs.

A person's body can adapt to cope with lower oxygen levels than are usual. However, in people with COPD, hypoxia in the lungs means oxygen levels become extremely low. When oxygen levels reach an extremely low level, the organs in the body do not get enough oxygen and start to develop damage and injury. This is known as hypoxemia.

Hypoxia and hypoxemia can also lead to other conditions, including neurocognitive dysfunction, pulmonary hypertension, secondary polycythemia, skeletal muscle dysfunction, and systemic inflammation, to name a few conditions.

Neurocognitive dysfunction. Hypoxia can cause injury to the brain and nerve cells. Hypoxia may trigger changes in brain function and thought processes. If COPD brings down brain oxygen levels too much, it can also reduce the number of neurotransmitters that the brain creates. Neurotransmitters are chemical messengers that ensure different parts of the brain and nervous system can communicate with each other. Sufficient oxygen is essential for creating these neurotransmitters and making sure they function correctly.

Pulmonary hypertension. Typically, the right side of the heart pumps low oxygen blood to the lungs. Once it reaches the lungs, blood can pick up oxygen from inhaled air. The oxygenated blood then returns to the left side of the heart, which pumps it throughout the body. A person with COPD can experience inflammation in the arteries that transfer blood from the right side of the heart to the lungs. This inflammation can cause high pressure to develop in these arteries, which is also known as pulmonary hypertension. This higher pressure means that the right side of the heart has to work harder to pump blood through those arteries. The over work of the heart can cause damage to the heart, making it weak.

Secondary polycythemia. Secondary polycythemia is the body's response to chronic hypoxia due to COPD. The body starts creating extra red blood cells to transport more oxygen. Extra blood cells mean that the blood is more prone to clotting. In people who have COPD, this condition can also increase the risk of abnormal heart rhythms, longer hospital stays, and more breathing complications.

Skeletal muscle dysfunction. People whose COPD has reached a more advanced stage can have difficulty engaging in physical exercise and activity. As a result, their muscles start to become weak. The weakened muscles are more easily fatigued, making it difficult for a person with COPD to exercise. Lack of exercise or the inability to exercise can exacerbate numerous conditions of the patient with COPD.

Systemic inflammation. A chronic lack of oxygen can cause inflammation in the body's tissues, which can lead to several conditions. Examples include atherosclerosis, which makes the arteries harden. Atherosclerosis increases the risk of heart disease, heart attack, and stroke.

If a person has severe hypoxemia due to COPD, a doctor will commonly recommend oxygen therapy. Oxygen therapy involves breathing in extra oxygen through a small, flexible tube, or nasal cannula, that fits in the nostrils. According to a 2019 study in the open access scientific journal PLOS One, long-term oxygen therapy may improve the quality of life for people with COPD hypoxemia by decreasing COPD flares and increasing tolerance to physical activity.

Current studies show that individuals with severe COPD are likely to benefit the most from oxygen treatment. However, not everyone with COPD is a good candidate for supplemental oxygen. It is advisable that a doctor carefully prescribes and closely monitors oxygen therapy. Too much oxygen can lead to higher carbon dioxide levels, although exercise can be challenging for people with COPD, physical activity can improve oxygen uptake in the lungs of a person with mild COPD and improve their breathing patterns. Those with more advanced hypoxia, however, may find any amount of physical activity difficult.

In addition to COPD, other diseases treated with supplemental oxygen include pulmonary fibrosis, pneumonia, cystic fibrosis, severe acute respiratory syndrome coronavirus (SARS-CoV-2 or COVID-19), and sleep apnea.

### Background Respiratory System

The average total lung capacity of a human male at sea level is about six liters (6L) while the same for a female is about four and two tenths liters (4.2L). Larger, more physically fit people and people living at higher altitudes typically have a larger total lung capacity. Tidal volume, the volume of air moved into or out of the lungs during quiet breathing for both sexes averages about five hundred milliliters (500ml).

Air is a mixture of gases, primarily nitrogen (N2; 78.6 percent), oxygen (O₂; 20.9 percent), water vapor (H₂O; 0.5 percent), and carbon dioxide (CO₂; 0.04 percent). In the body, oxygen is used by cells of the body's tissues and carbon dioxide is produced as a waste product. The ratio of carbon dioxide production to oxygen consumption is the respiratory quotient ("RQ"). RQ typically varies between seven tenths and one (0.7-1.0).

Each gas component of air mixture exerts a pressure. The pressure for an individual gas in a mixture is the partial pressure of that gas. As stated above, approximately twenty-one percent (21%) of atmospheric gas is oxygen. Carbon dioxide, however, is found in relatively small amounts, approximately four hundredths percent (0.04%). The partial pressure for oxygen is, therefore, much greater than that of carbon dioxide.

The RQ is used to calculate the partial pressure of oxygen in the alveolar spaces within the lung, the alveolar partial pressure of oxygen ("PO₂").

The structure of the lung maximizes its surface area to increase gas diffusion. Because of the enormous number of alveoli (approximately three hundred million (300,000,000) in each human lung), the surface area of the lung is very large, approximately seventy-five square meters (75 m²). Having such a large surface area increases the amount of gas that can diffuse into and out of the lungs.

In the lungs, oxygen diffuses out of the alveoli and into the capillaries surrounding the alveoli. Oxygen (about ninety-eight percent (98%)) binds reversibly to the respiratory pigment hemoglobin found in red blood cells (RBCs). RBCs carry oxygen to the tissues where oxygen dissociates from the hemoglobin and diffuses into the cells of the tissues. More specifically, alveolar PO₂ ("PₐₗᵥO₂") is higher in the alveoli (PₐₗᵥO₂ = 100 mm Hg) than in blood PO₂ (40 mm Hg) in the capillaries. Because this pressure gradient exists, oxygen diffuses down its pressure gradient, moving out of the alveoli and entering the blood of the capillaries where O₂ binds to hemoglobin. At the same time, alveolar partial pressure of carbon dioxide ("PCO₂") is lower PₐₗᵥO₂ = 40 mm Hg than blood PCO₂ = (45 mm Hg). CO₂ diffuses down its pressure gradient, moving out of the capillaries and entering the alveoli.

The result of breathing increased partial pressures of oxygen is hyperoxia, an excess of oxygen in body tissues. Hypercapnia is when a patient has too much carbon dioxide (CO₂) in their bloodstream. Excess CO₂ usually happens as a result of hypoventilation, or not being able to breathe properly and get oxygen into the lungs. Chronic Restrictive Diseases such as pulmonary fibrosis, Infant Respiratory Distress Syndrome and pneumothorax cause a reduction in lung volume capacity. Chronic Obstructive Diseases such as asthma, COPD, and emphysema do not result in decreased lung capacity, but flow rates are impeded.

The gas exhaled from a human is approximately four to five percent (4-5%) by volume of carbon dioxide, about a one hundred (100) fold increase over the inhaled amount. The volume of oxygen is reduced by a small amount, four to five percent (4-5%), compared to the oxygen inhaled. See table 1.

### Typical Exhalation

**Table 1**

| |
|---|
| • 5.0-6.3% water vapor |
| • 74.4% nitrogen |
| • 13.6-16.0% oxygen |
| • 4.0-5.3% carbon dioxide |
| • 1% argon |

Average lung volumes and capacities of an average adult male are as follows (Table 2)

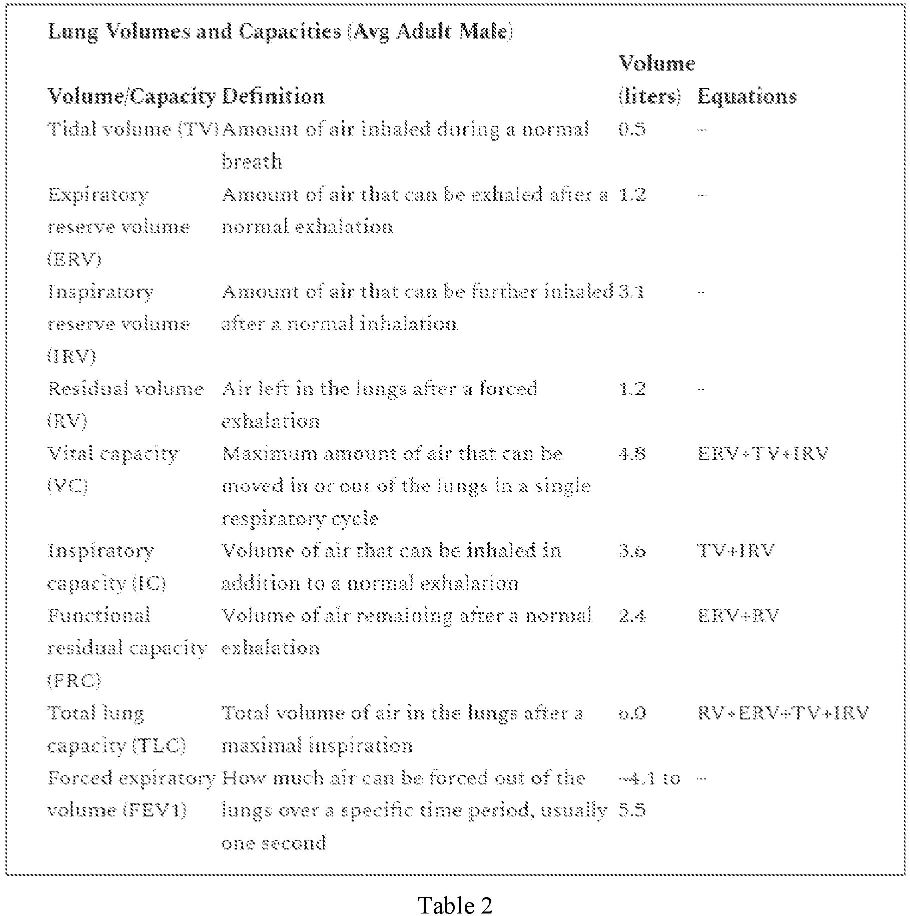

One reason a ventilator may be better than supplemental oxygen in raising a patient's blood oxygen saturation level has to do with a patient's inspiration abilities and their lung oxygen and carbon dioxide exchange/transfer effectiveness.

Schematic A shows a typical human pulmonary circuit, including partial pressures of oxygen and carbon dioxide in ambient air, arterial blood, and venous blood. If a patient has poor inspiration capabilities, elevating the oxygen concentration through supplemental oxygen improves oxygen and carbon dioxide exchange in the lungs but the patient will likely have difficulty exhaling carbon dioxide as the tidal volume will not be enough to provide the pressure needed for proper gas exchange. By Henry's law, the solubility of a gas in a liquid is directly proportional to the pressure of that gas above the surface of the solution. This applies to mechanical or noninvasive ventilation in that increasing positive end-expiratory pressure ("PEEP") increases the pressure in the system. This, in turn, increases the solubility of oxygen and its ability to cross the alveolocapillary membrane and increase the oxygen content in the blood.

Table 3 reflects measurements of oxygen concentration for patients with various tidal volumes at various supplemental oxygen levels.

**Table 3**

| **Fractional Oxygen Inspired (FiO₂₎** | | | | | | | | | | | | | | | | | |
|---|---|---|---|---|---|---|---|---|---|---|---|---|---|---|---|---|---|
| | | | | **Pulse** | | **Oxygen (LPM)** | | | | | | | | | | | |
| | **0** | **1** | | **2** | | **3** | | **4** | | **5** | | **6** | | | **Breaths Per Minute** | | **15** |
| **Tidal Volume (ml)** | **O₂ Norm (ml)** | **O₂ ml** | **O₂%** | **O₂ ml** | **O₂** % | **O₂ ml** | **O₂%** | **O₂ ml** | **O₂ %** | **O₂ ml** | **O₂ %** | **O₂ ml** | **O₂ %** | | | | |
| 600 | 126 | 12.7 | 22.6% | 25.3 | 24.1% | 38.0 | 25.6% | 50.7 | 27.0% | 63.3 | 28.4% | 76.0 | 29.7% | | **Oxygen Level - Air** | | **21%** |
| 550 | 115 | 12.7 | 22.7% | 25.3 | 24.4% | 38.0 | 26.0% | 50.7 | 27.5% | 63.3 | 29.0% | 76.0 | 30.4% | | | | |
| 500 | 105 | 12.7 | 22.9% | 25.3 | 24.7% | 38 0 | 26.4% | 50.7 | 28.1% | 63.3 | 29.7% | 760 | 31.2% | | **Oxygen level- Source** | | **95%** |
| 450 | 94 | 12.7 | 23.1% | 25.3 | 25.1% | 38.0 | 27.0% | 50.7 | 28.8% | 63.3 | 30.5% | 76.0 | 32.1% | | | | |
| 400 | 84 | 12.7 | 23.3% | 25.3 | 25.6% | 38.0 | 27.7% | 50.7 | 29.7% | 63.3 | 31.5% | 76.0 | 33.3% | | | | |
| 350 | 73 | 12.7 | 23.7% | 25.3 | 26.2% | 38.0 | 28.5% | 50.7 | 30.7% | 63.3 | 32.8% | 76.0 | 34.7% | | | | |
| 300 | 63 | 12.7 | 24.1% | 25.3 | 27.0% | 380 | 29.7% | 50.7 | 32.1% | 63.3 | 34.4% | 76.0 | 36.5% | | | | |
| 250 | 52 | 12.7 | 24.7% | 25.3 | 28.1% | 38.0 | 31.2% | 50.7 | 34.0% | 63.3 | 36.5% | 76.0 | 38.9% | | | | |

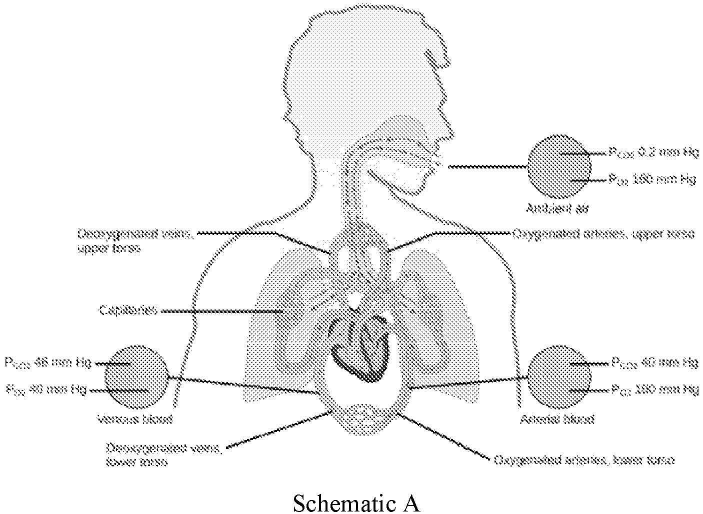

PEEP is a mode of therapy used in conjunction with mechanical ventilation. At the end of mechanical or spontaneous exhalation, PEEP maintains the patient's airway pressure above the atmospheric level by exerting pressure that opposes passive emptying of the lungs. This pressure is typically achieved by maintaining a positive pressure flow at the end of exhalation. This pressure may be measured in centimeters of water.

PEEP therapy can be effective when used in patients with a diffuse lung disease that results in an acute decrease in functional residual capacity ("FRC"), which is the volume of gas that remains in the lung at the end of a normal expiration. FRC is determined by primarily the elastic characteristics of the lung and chest wall. PEEP is typically utilized for acute respiratory failure syndrome ("ARFS") to allow reduction in the level of oxygen being given. Extrinsic PEEP can be used to increase oxygenation.

Extrinsic PEEP can also be used to improve ventilation-perfusion ("VQ") mismatches. The application of positive pressure inside the airways can open or "splint" airways that may otherwise be collapsed, decreasing atelectasis, improving alveolar ventilation, and, in turn, decreasing VQ mismatch.

The application of extrinsic PEEP will, therefore, have a direct impact in oxygenation and an indirect impact on ventilation. By opening up airways, the alveolar surface increases, creating more areas for gas exchange and somewhat improving ventilation. Nevertheless, extrinsic PEEP should never be used for the sole purpose of increasing ventilation. If a patient needs to clear CO₂ by improving ventilation, the patient should receive some level of pressure support for ventilation, either via bilevel positive airway pressure ("BPAP") or invasive ventilation.

Extrinsic PEEP also significantly decreases the work of breathing. This is especially important for stiff lungs with low compliance. In intubated patients with low compliance, work of breathing can represent an important part of their total energy expenditure (up to thirty percent (30%)). This increases CO₂ and lactate production, both of which may be problems of their own. By decreasing work of breathing, CO₂ and lactate production decreases, decreasing the need for high minute ventilation (to correct the hypercapnia and acidosis) and thereby decreasing respiratory drive and further decreasing the work of breathing needed by the patient in a positive-effect loop. An article by Carpio titled, "Positive End-Expiratory Pressure" describes PEEP and reviews the indications, contraindications, complications and other elements of the use of PEEP related to the care of patients requiring ventilation.

The use of extrinsic PEEP is tricky, and although it may be beneficial, it has to be guided by good clinical sense. In cases of dynamic flow obstruction, especially in COPD where there is alveolar collapse, (Note: This does not include asthma where there is inflammation of the airway but not necessarily airway collapse.) the application of extrinsic PEEP will splint the airways open, permitting this way for air to be flushed from alveolar pouches and decreasing auto-PEEP. Extrinsic PEEP will also decrease work of breathing in the appropriate setting. Nevertheless, when applied in the wrong setting, like in patients without dynamic airflow obstruction, the application of extrinsic PEEP will generate back pressure that will prevent air from getting out of the lungs, worsening air trapping. It is also important to understand that the extrinsic PEEP initially will add to the auto-PEEP, increasing the intrathoracic pressure. When used, it is recommended to maintain extrinsic PEEP below seventy-five to eighty-five percent (75-85%) of the auto-PEEP. Again, the use of extrinsic PEEP to treat auto-PEEP is driven by strong clinical sense as not all patients will benefit from it and others will be harmed. A practical way to assess the effects of extrinsic PEEP in auto-PEEP is to apply small increments of extrinsic PEEP and check the static pressures in the lungs. If the static pressures do not increase, then applying extrinsic PEEP may benefit the patient, but if the pressures increase, then it is time to back down on this strategy.

Another factor in designing respiratory treatment devices is understanding that breathing is dynamic, both breath rate and duration change with activities. Oxygen and tidal volume assist are typically correlated to the patient's requirements. Table 4 shows a typical COPD patient's breathing patterns.

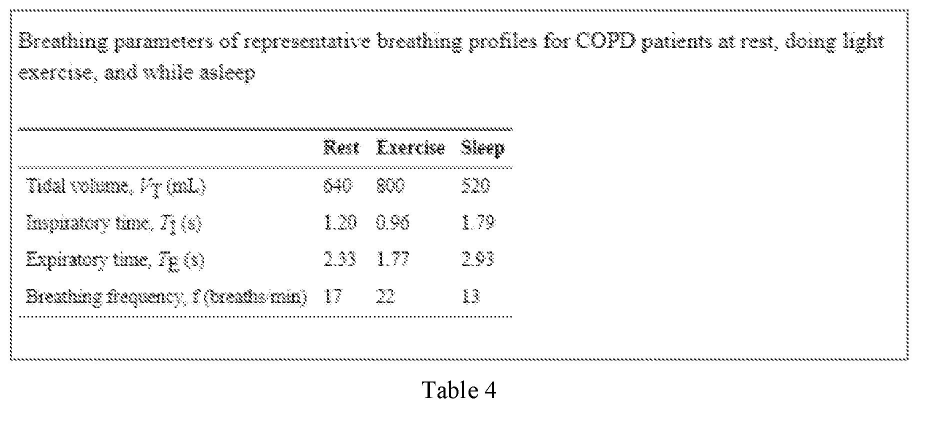

When treating a patient needing supplemental oxygen it is important to understand the different blood oxygen saturation levels required for non-COPD patients compared to COPD patients. For non-COPD patients, the respiratory therapist should aim for a saturation between ninety-four to ninety-eight percent (94-98%). Contrary to what may have been practiced in the past, too much oxygen can be harmful. If a patient has a saturation level of one hundred percent (100%) oxygen, the amount of oxygen should be decreased as it creates to high partial pressure of oxygen.

Patients with COPD should have eighty-eight to ninety-two percent (88-92%) saturation levels as their target. The reason to target lower oxygen saturation levels is because COPD patients typically retain too much CO₂. Reasons for retaining CO₂ include severe obstructive lung disease such as bronchiectasis or cystic fibrosis or severe restrictive lung diseases such as those caused by neuromuscular diseases, severe kyphoscoliosis and severe obesity.

Patients with COPD optimize their gas exchange by hypoxic vasoconstriction leading to altered alveolar ventilation-perfusion ratios ("V/Q"). Excessive oxygen administration overcomes this, leading to increased blood flow to poorly ventilated alveoli, increasing the V/Q mismatch and therefore increasing physiological dead space. This increase in V/Q mismatch occurs in both CO₂ retainers and non-retainers but the difference seems to be more profound in certain patients. Another reason too much oxygen can be bad for COPD patients is due to the Haldane effect. Deoxygenated hemoglobin (Hb) binds with CO₂ with greater affinity than oxygenated hemoglobin (HbO₂). Oxygen, therefore, induces a right shift of the CO₂ dissociation curve, which is called the Haldane effect. In patients with severe COPD who cannot increase minute ventilation, the Haldane effect accounts for about twenty-five percent (25%) of the total PaCO₂ increase due to O₂ administration.

### Oxygen Delivery

One way to deliver increased oxygen flow and pressure to create PEEP while not providing too much oxygen is to use a venturi mask or nasal cannula.

Typical low-flow oxygen systems provide supplemental oxygen often less than the patient's total minute ventilation. Because the patient's minute ventilation exceeds flow, the oxygen delivered by the device will be diluted with ambient air and thus the inspired oxygen delivery is less than anticipated. Low-flow oxygen delivery systems consist of nasal cannula, nasal catheters, and transtracheal catheters.

The standard nasal cannula delivers an FiO₂ of twenty-four to forty-four percent (24-44%) at supply flows ranging from one to eight liters per minute (1-8 LPM). A general formula is FiO₂ = twenty percent (20%) + (4 × oxygen liter flow). The FiO₂ is influenced by breath rate, tidal volume and pathophysiology. The slower the inspiratory flow, the higher the FiO₂; the faster the inspiratory flow, the lower the FiO₂. Since the delivered oxygen percentage is very inconsistent during respiratory distress, a nasal cannula is not recommended for acute severe hypoxemia or patients that breathe on a hypoxic drive where too high of an oxygen concentration may lead to respiratory depression. A nasal cannula utilizes no external reservoir of oxygen and relies on the patient's upper airway as an oxygen reservoir. A humidification device is recommended for flows greater than about four liters per minute (4 LPM) to ensure humidification of the dry inspired gas. Even with humidity, added flows of six to eight liters per minute (6-8 LPM) can cause nasal dryness and bleeding. The best clinical indications for the nasal cannula are for patients who have a relatively stable respiratory pattern, who require low oxygen percentage, or who need supplemental oxygen during an operative or diagnostic procedure, or for chronic home care.

A nasal catheter is a soft paste tube with several holes at the tip. The nasal catheter is inserted into a nare or nostril, which typically needs to be changed every eight hours. The nasal catheter device has been replaced by the nasal cannula in certain situations but can be used for a patient that is undergoing an oral or nasal procedure.

Transtracheal catheters deliver oxygen directly into the trachea. There are washout and storage effects that promote gas exchange, as well as provide high-flow oxygen. High-flow transtracheal catheters may reduce the work of breathing and augment CO₂ removal in the chronic oxygen user. Transtracheal oxygen therapy improves the efficiency of oxygen delivery by creating an oxygen reservoir in the trachea and larynx. Consequently, mean oxygen savings amount to approximately fifty percent (50%) at rest and thirty percent (30%) during exercise. Transtracheal oxygen reduces dead space ventilation and inspired minute ventilation while increasing alveolar ventilation slightly, which may result in a reduction of the oxygen cost of breathing. As a result, patients using this device may experience improved exercise tolerance and reduced dyspnea. This delivery device is best used for home care and ambulatory patients who require long periods of mobility and do not feel comfortable wearing a nasal cannula.

Air Entrainment Devices include venturi masks and nasal cannulas. With a traditional Venturi mask system, oxygen inflow is connected to a specific color-coded entrainment device at the base of the mask that provides a set oxygen inflow rate. Various entrainment devices can provide a flow of oxygen from twenty-four hundredths to one-half (0.24-0.5), with an oxygen inflow of four to fifteen liters per minute (4-15 L/min) and a total flow delivered to the patient (including entrained air) of thirty-five to forty-five liters per minute (35-45 L/min). If the patient's peak inspiratory flow exceeds this total flow, a lower flow of oxygen is inspired. A venturi mask can be very beneficial for a patient with COPD who has a low to moderate oxygen requirement but is at risk for hypercarbia with uncontrolled oxygen therapy.

Schematic B shows the fluid dynamics of a venturi device. For a venturi mask or cannula, the gas source is concentrated oxygen, typically greater than ninety-five percent (> 95%) oxygen purity. The negative pressure caused by the accelerating oxygen passing through the gas injector and into the larger cannula "pulls" gas, in this case, ordinary air through the narrowed cannula. The effect being a larger volume of "mixed" air and oxygen is delivered to the patient.

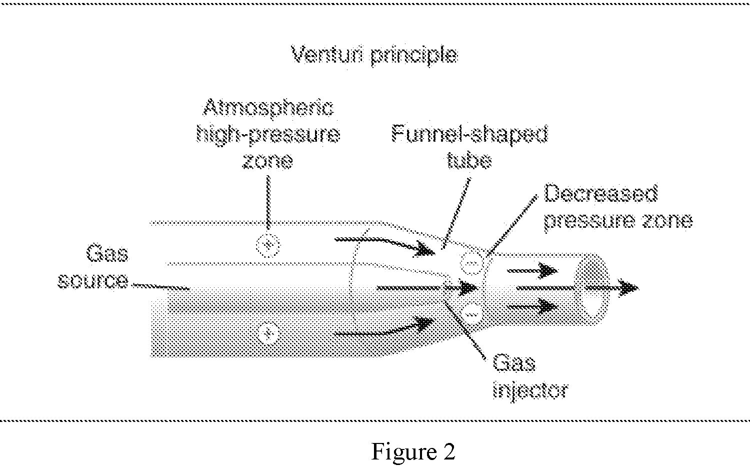

Currently, different colored venturis correspond to different flow rates. Blue is typically used with a flow rate of two to four liters per minute (2-4 L/min) and delivers approximately twenty-four percent (24%) O₂. White typically corresponds to four to six liters per minute (4-6 L/min) and twenty-eight percent (28%) O₂. Yellow typically corresponds to eight to ten liters per minute (8-10 LP/min) and thirty-five percent (35%) O₂. Red typically corresponds to ten to twelve liters per minute (10-12 L/min) and forty percent (40%) O₂ and Green typically corresponds to twelve to fifteen liters per minute (12-15 L/min) and sixty percent (60%) O₂.

### Non-portable ventilation

Prior art ventilators, except as described below, are designed for hospital or home use with little to no consideration given for portability as the patient is typically not mobile and the equipment requires a stationary power source, *i.e*., wall socket or is extremely heavy and cumbersome even if a portable power source could be utilized.

Two pseudo portable ventilators are described herein. The first is the VOCSN by Ventec Life Systems. The VOCSN is a combination ventilator, supplemental oxygen, suction, cough assist and nebulizer device that weighs over eighteen pounds (18 lbs.). The VOCSN claims to be portable, but it is hardly wearable and can be difficult for patients with breathing issues to transport.

The second device is the Life2000 ventilation system by Breathe Technologies. The Life2000 ventilator claims to be only one pound (1 lb.) and is wearable, however, the Life2000 ventilator must be connected to an oxygen source or compressor in order to provide ventilation to a patient. The Life2000 compressor is powered via a 110/220 power outlet. Alternatively, an oxygen metal cylinder can be used as a source of oxygen to drive the ventilator to provide oxygen or mixed air and oxygen to a patient. The oxygen cylinder, unlike an oxygen concentrator has a finite supply of oxygen, can be relatively heavy and creates a potential combustion hazard.

Ventilators contribute energy to the respiratory system in the form of pressure assist and/or increased oxygen content of the breathing air. Physicians often prescribe both pressure assist and oxygen therapy together.

Various oxygen delivery systems are known, e.g., from US 4,971,609 A, WO 2017/205542 A1, WO2016/174380 A1, FR 2 727 023 A1, US 2015/083121 A1, US 2014/283834 A1 and WO 2019/221852 A1. Specifically, US 2019/175856 A1 discloses an oxygen delivery system for delivering continuous positive airway pressure in conjunction with oxygen delivery. The system comprises: a controllable flow generator operable to provide breathable gas at a first pressure, wherein said first pressure is above atmospheric pressure; a gas delivery tube coupled to said controllable flow generator; a controllable oxygen concentrator device operable to provide oxygen-enriched gas; an enriched gas delivery tube coupled to said controllable oxygen concentrator device and connected to said controllable flow generator; a patient mask coupled to said gas delivery tube to receive breathable gas from said flow generator and to provide said gas, at a treatment pressure, to an airway of a patient; a controller operable to receive an input signal and to control the magnitude of said treatment pressure provided by said flow generator; and sensor to detect patient respiratory airflow and to generate an input signal to said controller. The controllable flow generator comprises a blower. The controllable oxygen concentrator device comprises at least one user-replaceable sieve cartridge comprising less than about 50 g adsorbent, and a linear compressor.

### SUMMARY OF THE INVENTION

The present invention provides an oxygen delivery system for delivering concentrated oxygen to a patient as defined in claim 1. Further preferred embodiments of the present invention are defined in the dependent claims.

The present invention with the features of claim 1 seeks to address the deficiencies of prior art devices to provide portable ventilation to patients that require supplemental oxygen and prefer to maintain mobility, which often has beneficial effects for the patient.

### SUMMARY OF THE PRESENT DISCLOSURE

The preferred embodiment of the present disclosure combines oxygen delivery functions into a portable and preferably carriable unit, even more preferably carriable by a patient with a breathing ailment. The preferred unit or system is preferably carriable by ordinary or average patients with COPD or other lung diseases and preferably allows the patient mobility. A lightweight, preferably less than eight pound (8 lb.), portable ventilator device or system is designed for portability and to provide concentrated oxygen to the patient. To be portable the ventilator is battery operated and able to generate its own oxygen. In addition, the preferred device is scalable and configurable to decrease the weight and cost of the device to the patient, as their disease progresses, or oxygen needs change due to exercise or environment.

Briefly stated, the preferred device is directed to a portable oxygen delivery system including an oxygen concentrator having a housing, a compressor mounted inside the housing, a removably mounted cartridge located within the housing and in fluid connection with the compressor, the cartridge containing a zeolite for removing Nitrogen from air through a pressure swing adsorption process for creating concentrated oxygen, a power source attached to the housing and an oxygen controller device for electronically controlling the pressure swing adsorption process. The portable oxygen delivery system also preferably includes a blowing apparatus fluidly connected to the oxygen concentrator having a blower housing, a blower motor mounted inside the blower housing, a blower fan connected to the blower motor, a second power source attached to the blower housing and a blower controller device for electronically controlling the blower.

The preferred system may be utilized with portable oxygen concentrators described in US Patent Nos. 8,888,902; 8,894,751; 9,199,055; 9,492,781; and 9,993,765, as well as International Patent Application Publication No. WO 2018/226532. These portable oxygen concentrators may be employed with the preferred system described therein to provide a source of portable concentrated oxygen to address the patient's COPD or other breathing disease.

### BRIEF DESCRIPTION OF THE SEVERAL VIEWS OF THE DRAWINGS

The foregoing summary, as well as the following detailed description of preferred embodiments of the device, system and method of the present disclosure, will be better understood when read in conjunction with the appended drawings. For the purposes of illustrating the portable ventilator, system and method, there are shown in the drawings preferred embodiments. It should be understood, however, that the disclosure is not limited to the precise arrangements and instrumentalities shown. In the drawings:
Fig. 1 is a side perspective view of an oxygen concentrator and an enlarged, partial cross-sectional view of a sieve module taken from within the dashed oval that may be utilized with a preferred portable ventilator system of the present disclosure;
Fig. 2A is a top perspective view of a portable ventilator in accordance with a first preferred embodiment of the present disclosure, including an oxygen concentrator system with an oxygen concentrator, a remote flow control or a blowing apparatus and a patient interface, preferably a nasal cannula with portions of the remote flow control or blowing apparatus being transparent to illustrate certain components;
Fig. 2B is an alternative top perspective view of the portable ventilator system of Fig. 2A, wherein portions of the remote flow control or blowing apparatus are transparent to illustrate certain components;
Fig. 2Ba is a partial top perspective view of an oxygen concentrator and a remote flow control or blowing apparatus with integrated blower of the portable ventilator system of Fig. 2A, wherein the blowing apparatus is enlarged, and portions are transparent to illustrate certain components;
Fig. 2Bb is a partial top plan and front elevational view of a supplemental oxygen delivery system having an oxygen cylinder and a remote blowing apparatus with integrated blower in accordance with a second preferred embodiment, wherein the remote flow control or blowing apparatus is enlarged, and portions are transparent to illustrate certain components;
Fig. 2C is a top perspective view of an oxygen concentrator system with an integrated blower, flow control system and patient interface in accordance with a third preferred embodiment, wherein portions of the oxygen concentrator are transparent to show internal components;
Fig. 3 is a graph of gas pressure and flow for one of the preferred systems;
Fig. 4 are top perspective and front elevational views of the portable ventilator system of Fig. 2A, wherein the system is shown individually and mounted to a patient and the system is preferably configured for producing eight liters per minute (8 L/min) of pulse dose oxygen flow;
Fig. 5 are front elevational and top plan views of a ventilator control and delivery reservoir or blowing apparatus in accordance with any of the preferred embodiments;
Fig. 6 are alternative front elevational and top plan views of a ventilator control and delivery reservoir or blowing apparatus in accordance with any of the preferred embodiments;
Figs. 6A-6D are various detailed views of a remote flow control with integrated blower incorporating a medicant delivery apparatus in accordance with any of the preferred embodiments;
Fig. 7 is a perspective view of a portable ventilator system in accordance with the preferred embodiments of the present disclosure, wherein portions of the system are transparent to illustrate internal components;
Fig. 8 is a plan view illustration focusing on a patient interface with nasal air entrainment of a portable ventilator system in accordance with the preferred embodiments of the present disclosure;
Fig. 9 is a process flow diagram for utilization with the portable ventilator system of the preferred embodiments, representing a control system and feedback;
Fig. 10 is schematic illustration of a reservoir and a patient interface of a portable ventilator system in accordance with the preferred embodiments, wherein the portable ventilator is powered by a medium pressure oxygen source and portions of the system are transparent to illustrate internal components;
Fig. 11 is a graph of typical turbine or blower fan performance related to airflow and pressure for the portable ventilator system of the preferred embodiments;
Figs. 12A and 12B are top perspective, front elevational and top plan views of a blowing apparatus of a portable ventilator in accordance with a sixth preferred embodiment, wherein portions of the blowing apparatus of Fig. 12A are transparent to illustrate internal components of the reservoir;
Figs. 13A and 13B are top perspective, front elevational and top plan views of a top panel of the blowing apparatus of Figs. 12A and 12B, wherein portions of the top panel of Fig. 13A are transparent to illustrate internal components of the top panel;
Figs. 14A and 14B are top perspective, front elevational and top plan views of a blowing apparatus and related internal components of a portable ventilator in accordance with an alternative preferred embodiment of the portable ventilator system, wherein portions of the blowing apparatus of Fig. 14A are transparent to illustrate internal components of the blowing apparatus; and
Fig. 15A and 15B are top perspective, front elevational and top plan views of a removable battery component of the reservoir of Figs. 14A and 14B and related internal components of the removable battery component of Figs. 14A and 14B, wherein portions of the removable battery component of Fig. 15A are transparent to illustrate rechargeable batteries of the preferred reservoir.

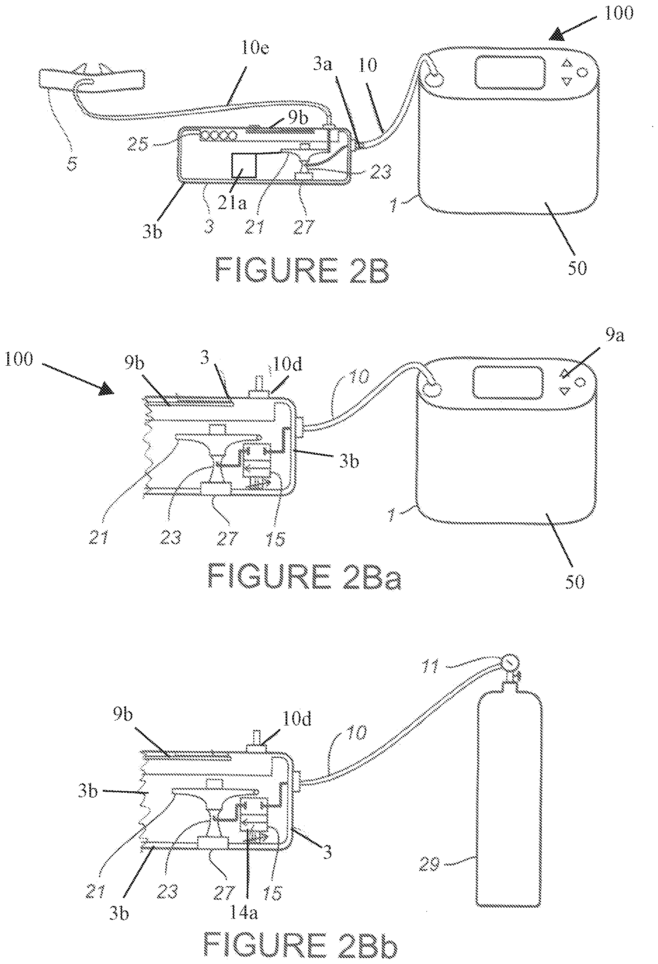

Figures 2B and 2Ba illustrate an oxygen delivery system (100) for delivering concentrated oxygen to a patient according to a preferred embodiment of the present invention (the scope of which is defined by the claims appended hereto).

### DETAILED DESCRIPTION OF THE DISCLOSURE

Certain terminology is used in the following description for convenience only and is not limiting. Unless specifically set forth herein, the terms "a", "an" and "the" are not limited to one element but instead should be read as meaning "at least one". The words "right," "left," "lower" and "upper" designate directions in the drawings to which reference is made. The words "inwardly" or "distally" and "outwardly" or "proximally" refer to directions toward and away from, respectively, the patient's body, or the geometric center of the preferred portable ventilator system and related parts thereof. The words, "anterior", "posterior", "superior," "inferior", "lateral" and related words and/or phrases designate preferred positions, directions and/or orientations in the human body to which reference is made and are not meant to be limiting. The terminology includes the above-listed words, derivatives thereof and words of similar import.

It should also be understood that the terms "about," "approximately," "generally," "substantially" and like terms, used herein when referring to a dimension or characteristic of a component of the preferred disclosure, indicate that the described dimension/characteristic is not a strict boundary or parameter and does not exclude minor variations therefrom that are functionally the same or similar, as would be understood by one having ordinary skill in the art. At a minimum, such references that include a numerical parameter would include variations that, using mathematical and industrial principles accepted in the art (e.g., rounding, measurement or other systematic errors, manufacturing tolerances, etc.), would not vary the least significant digit.

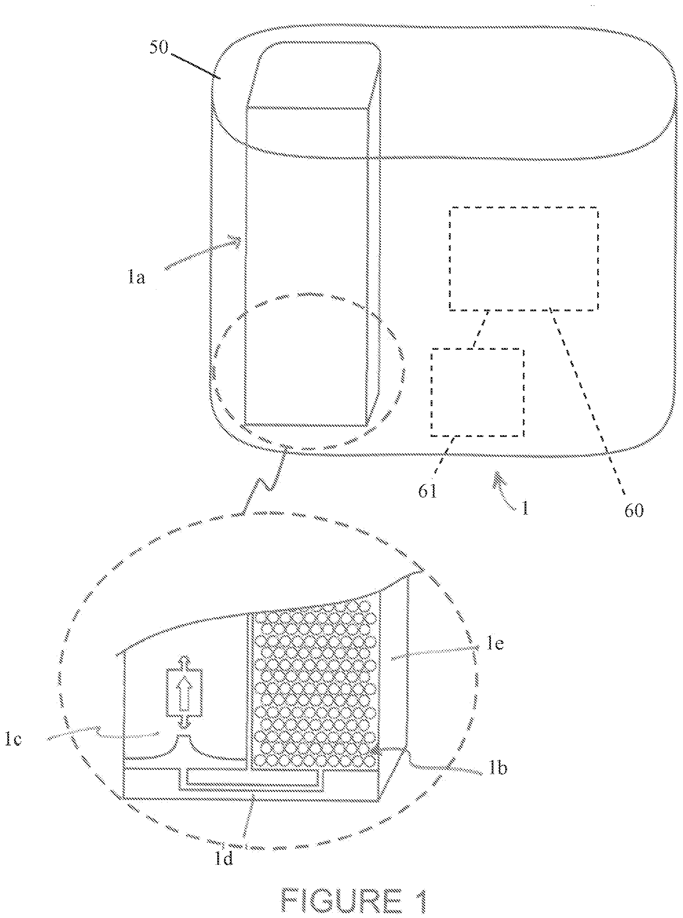

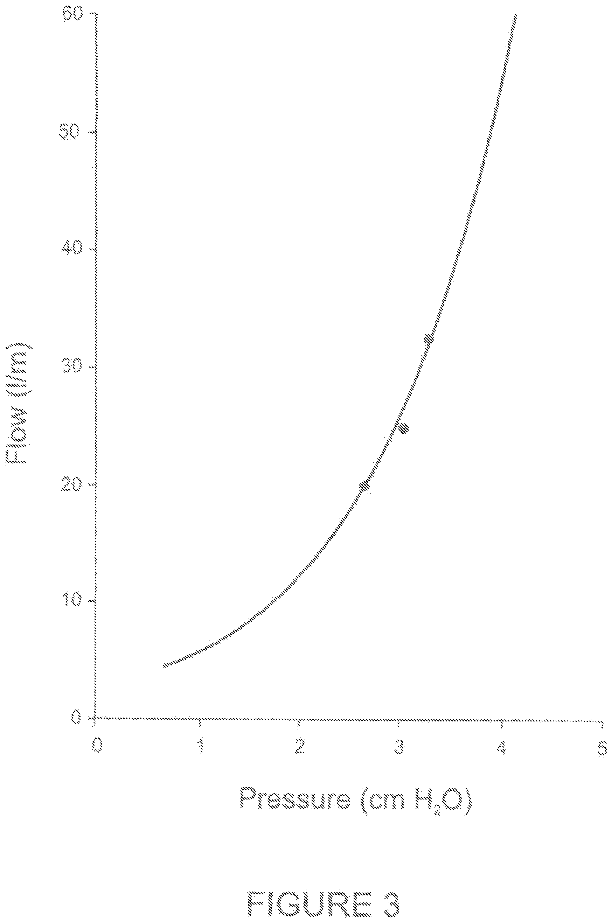

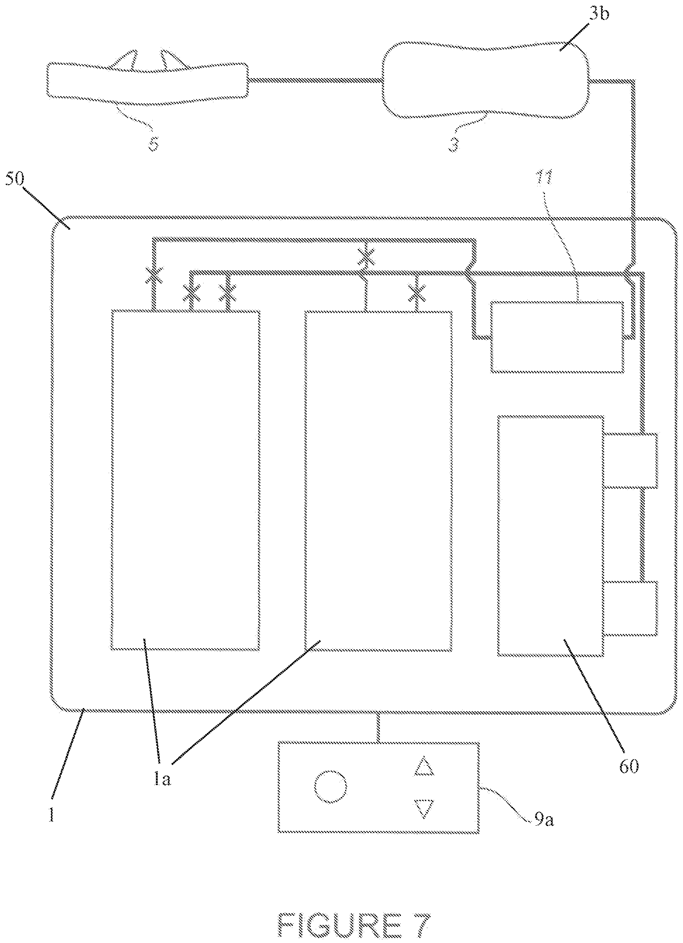

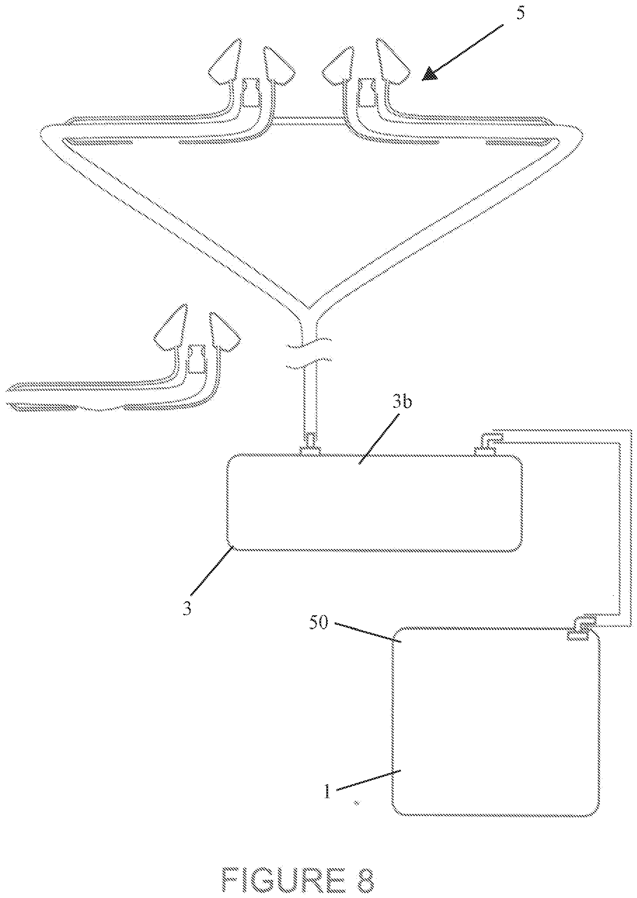

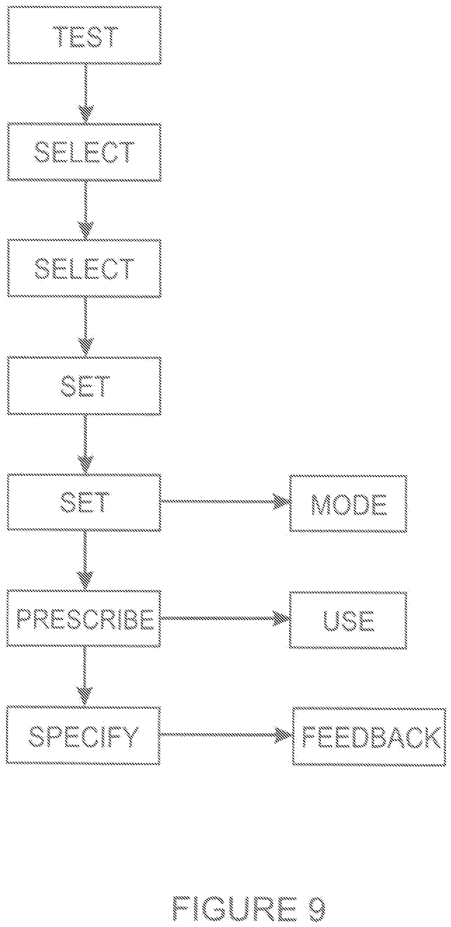

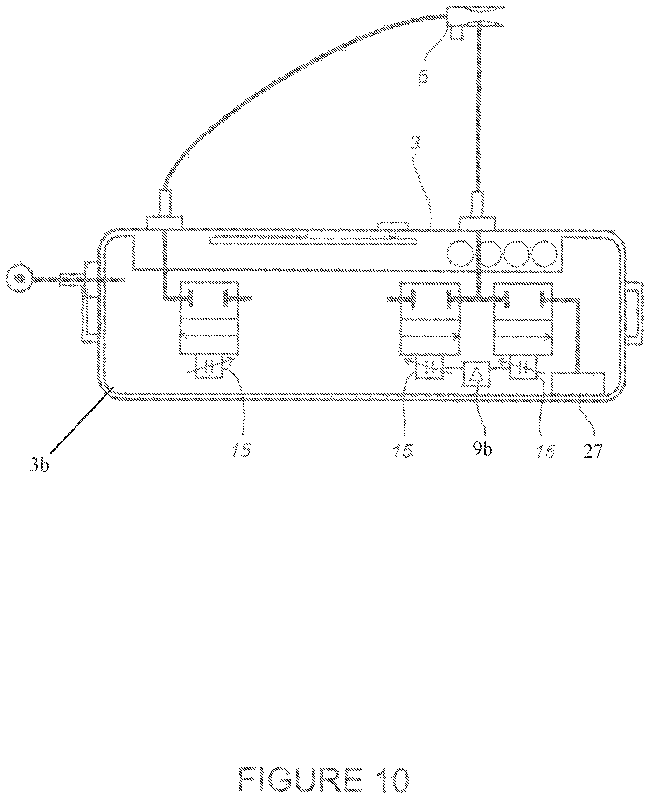

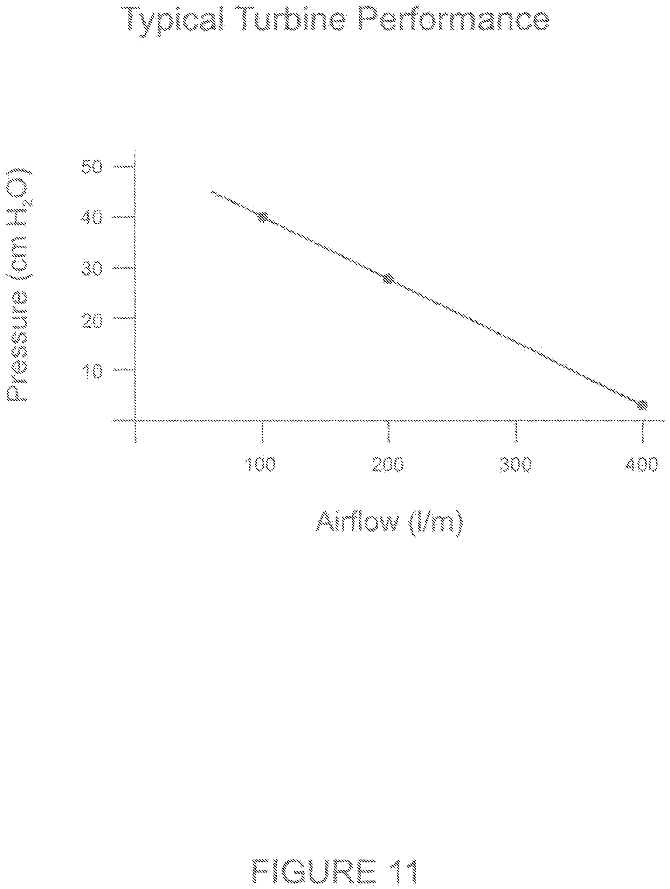

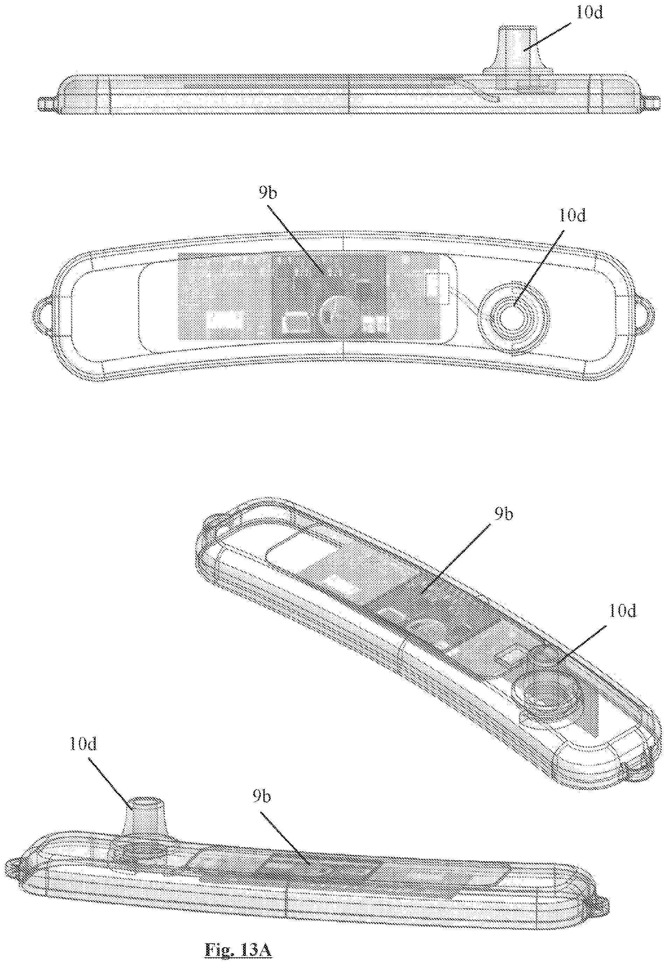

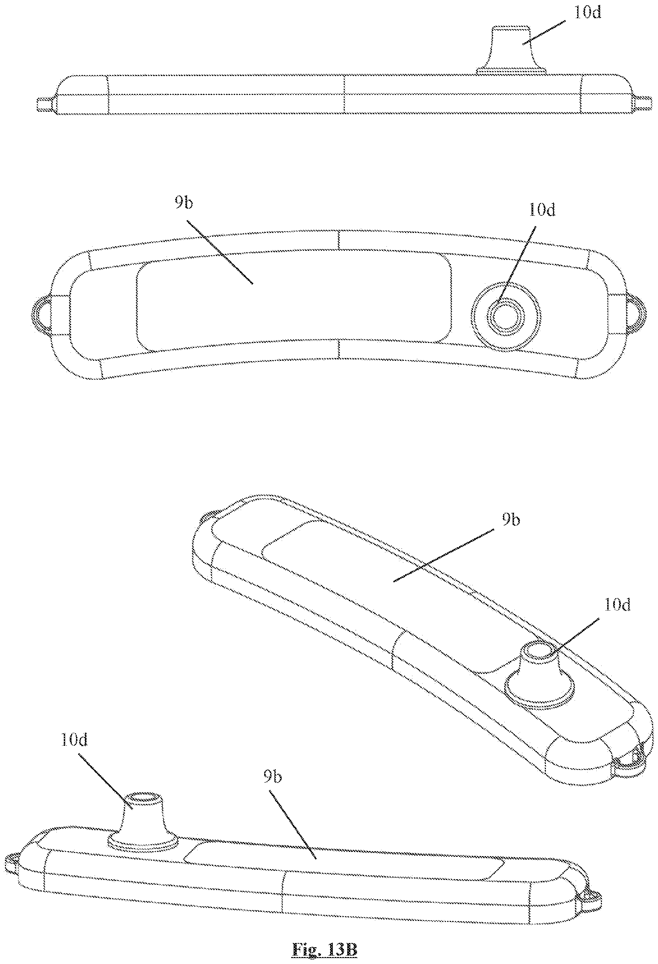

Referring to Figs. 1-15B, the preferred present disclosure is directed to multiple embodiments of a portable ventilator system and related method.

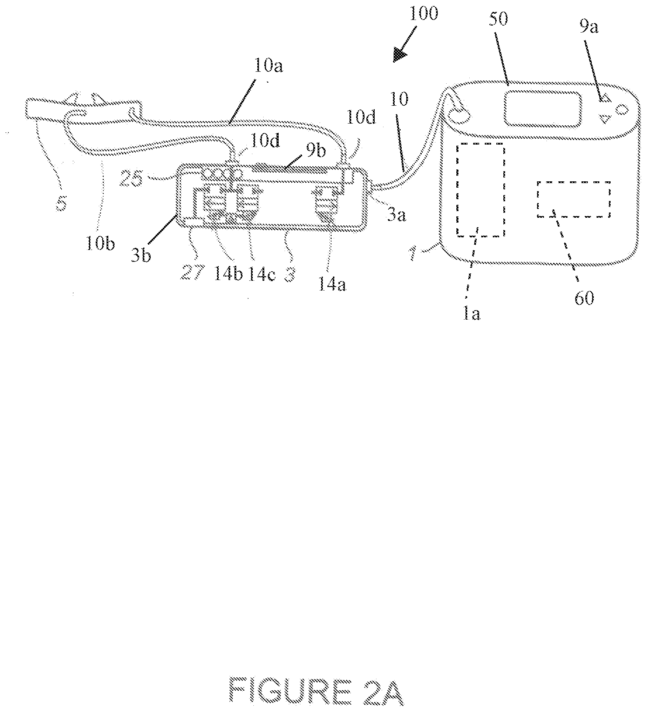

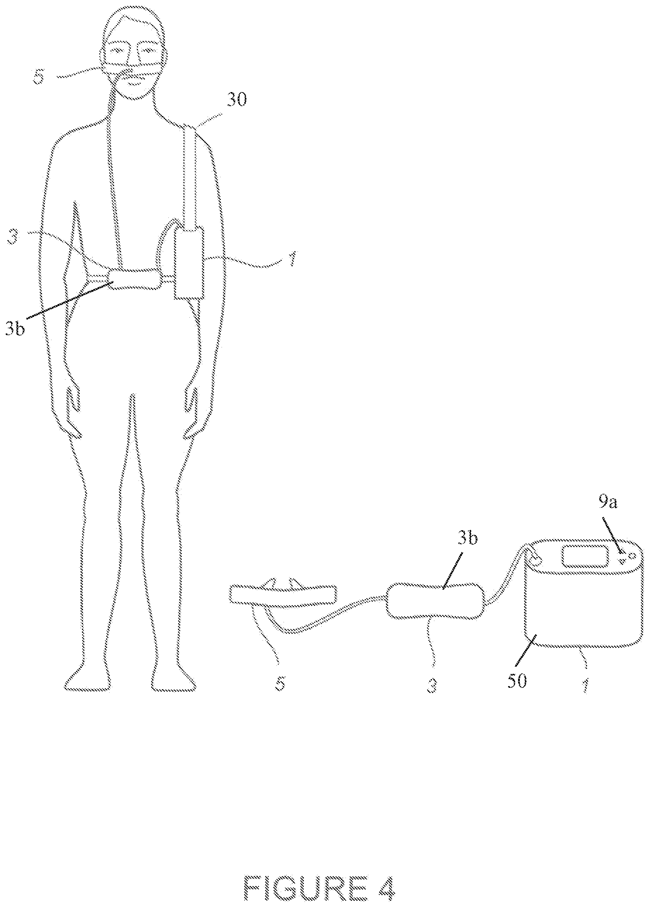

Referring to Figs. 2A-2Ba and 4, in a first preferred embodiment, an oxygen delivery system or portable ventilator, generally designated 100, for delivering concentrated oxygen to a patient utilizes a high-pressure air or oxygen and air source that increases flow to a patient through a venturi air induction system. Ordinary air or concentrated oxygen and air is generated by a portable oxygen concentrator such as one described in International Patent Application Publication No. WO 2018/226532, entitled Configurable Oxygen Concentrator and Related Method ("WO-532"). WO-532 is battery powered, weighs less than four pounds and can deliver one liter per minute if continuous flow oxygen or five liters per minute equivalent in pulse delivery mode. The oxygen concentrator of WO-532 can produce varying oxygen purities and flows. At low oxygen purity, for example forty to fifty percent (40-50%) oxygen, the flow can be as much as eight liters per minute (8 L/min) pulse dose. When operated at ten to twenty pounds per square inch (10-20 psig) outlet pressure, a venturi can induce a low-pressure airflow of up to two times the low concentrated oxygen air output flow. This is similar to the LifeAir 2000 system when a separate oxygen cylinder is integrated into the system. Higher capacity concentrators are also included in the design specifications for patients needing higher flows, pressures, or oxygen purity.

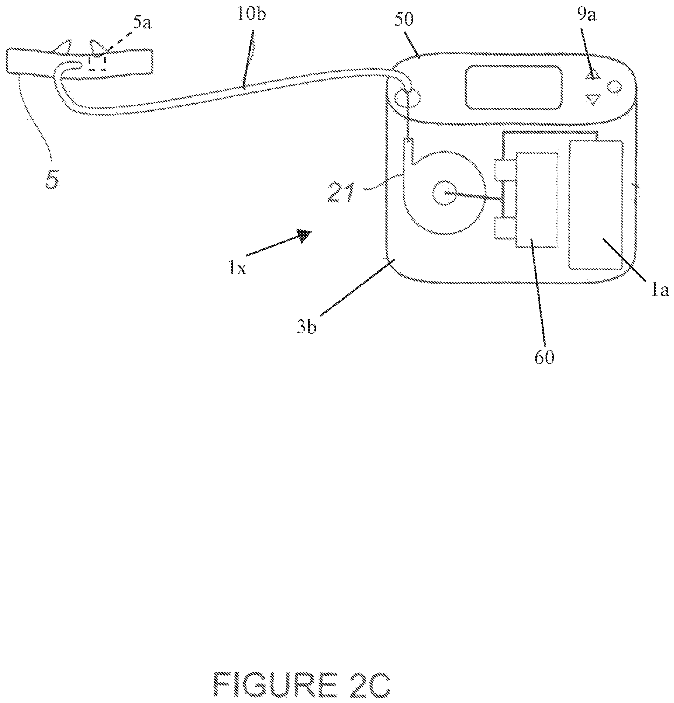

The basic design of the first preferred portable ventilator system 100 includes a portable oxygen concentrator 1, an air and oxygen flow controller or blowing apparatus 3 and a patient interface 5. The air and oxygen controller or blowing apparatus 3 can be remote to the portable oxygen concentrator 1, as shown in the embodiment of Figure 2A, or can be integrated into the oxygen concentrator 1, as I shown in the embodiment of Fig. 2C. The oxygen controller or blowing apparatus 3 preferably includes a blower housing 3b that encloses components for controlling the flow of oxygen to the patient interface 5. The blowing apparatus 3 preferably includes a blower controller device 9b for electronically controlling the blowing apparatus 3. The air and oxygen controller or blowing apparatus 3 preferably contains valves 14a, 14b, 14c that are controlled by the blower controller device 9b to control oxygen and air flow to the patient interface 5. A battery or batteries 25 preferably power the oxygen controller or blowing apparatus 3 and are preferably rechargeable. The valves 14a, 14b, 14c can be mechanically controlled or electronically controlled, preferably electronically controlled by the blower controller device 9b. It is also contemplated that control of the valves 14a, 14b, 14c could be wireless via Bluetooth or similar non-tethered systems or mechanisms. It is further contemplated that the oxygen controller or blowing apparatus 3 could be controlled via web or cellular connection, wherein a remote user could use a laptop or cell phone app to control the oxygen controller or blowing apparatus 3.

Referring to Fig. 1, an oxygen concentrator 1 that may be utilized with the portable ventilator 100 includes a removable sieve module 1a removably mounted within a housing 50 and a compressor 60 to compress ambient air for introduction into the sieve module 1a. The compressor 60 is preferably powered by a power source 61 attached to the housing 50, wherein the power source 61 is preferably comprised of a removable and rechargeable battery or batteries 61. The power source 61 is not limited to being comprised of removable and rechargeable batteries 61 and may be comprised of nearly any power source that is able to power the portable oxygen concentrator 1, specifically the compressor 60, such as an AC power connector. As described in WO-532 and further described herein, the sieve module or cartridge 1a comprises at least one cylinder 1e containing a zeolite material 1b. In general, the zeolite material 1b is comprised of, but not limited to, microporous aluminosilicate materials used to separate molecules through a process called adsorption. The cartridge or sieve module 1a is preferably removably mounted and located within the housing 50 and is in fluid connection with the compressor 60. The cartridge 1a contains the zeolite material 1b for removing nitrogen from ambient air through a pressure swing adsorption process for creating concentrated oxygen. The pressure swing adsorption process is preferably controlled by the oxygen controller 9a.

The zeolite material 1b is used as an ion-exchange bed in domestic and commercial water purification, softening, and other applications. In chemistry, the zeolite material 1b is used to separate molecules (only molecules of certain sizes and shapes can pass through), and as traps for molecules so they can be analyzed. The zeolite materials 1b are also widely used as catalysts and sorbents. Their well-defined pore structure and adjustable acidity make them highly active in a large variety of reactions. The zeolite materials 1b have the potential of providing precise and specific separation of gases, including the removal of water (H₂O), carbon dioxide (CO₂) and sulfur dioxide (SO₂) from low-grade natural gas streams. Other separations include noble gases, nitrogen (N₂), oxygen (O₂), freon and formaldehyde. As described in WO-532, zeolites are used to remove Nitrogen through a pressure swing adsorption ("PSA") process. A high-pressure valve 1c is shown at a distal end of a concentrated oxygen conduit 1d extending from the cylinder 1e of the sieve module 1a. Through the PSA process, ordinary air has Nitrogen molecules temporarily bonded to the zeolite material 1b and then evacuated from the cylinder 1e. The remaining concentrated oxygen is forced out of cylinder 1e into the tube 1d.

Referring to Fig. 2A a concentrated oxygen delivery tube 10 is in fluid connection between the oxygen concentrator 1 and the blowing apparatus 3, which is controlled by the blower controller device 9b. The blowing apparatus 3 stores pressurized oxygen for delivery to a patient interface 5 through a patient delivery hose 10e. The reservoir or blowing apparatus 3 includes the blower housing 3b that contains or houses components of the blowing apparatus 3. The blowing apparatus 3 is fluidly connected to the compressor 60 and includes the blower housing 3b, a blower motor 21a mounted inside the blower housing 3b, a blower fan 21 connected to the blower motor 21a, a second power source 25, preferably comprised of rechargeable batteries 25, attached to the blower housing 3b and a blower control device 9b for electronically controlling the blowing apparatus 3. A first valve 141, preferably a Piezo valve 14a, although not limited to a Piezo valve 14a and may be comprised of nearly any type or variety of valve to block and/or control the flow of oxygen or other gases and regulates concentrated oxygen from the oxygen concentrator 1 via the oxygen delivery tube 10a to the patient interface 5. A second valve 14b, also preferably Piezo valve 14b but not so limited, regulates ordinary air received through an inlet filter 27 that can be mixed with the concentrated oxygen received through a third valve 14, preferably also a Piezo valve 14c but not so limited, which is fluidly connected to the pressurized reservoir 3. The blended air and oxygen mixture is delivered through a patient delivery tube 10b to the patient interface 5.

Referring to Fig. 2Bb, in a second preferred or alternative preferred embodiment, the oxygen concentrator 1 is replaced with a cylinder 29 filled with compressed oxygen. The cylinder 29 is able to similarly provide concentrated oxygen to the reservoir 3.

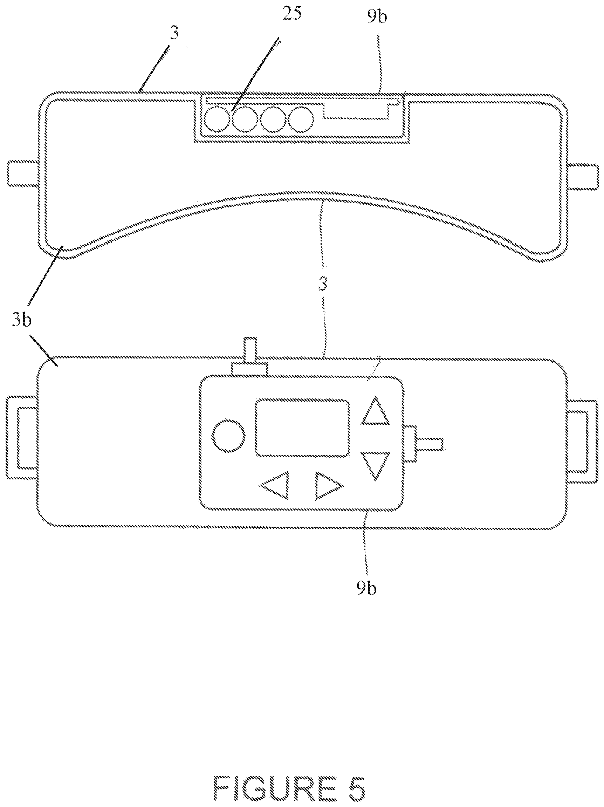

Referring to Figs. 2A-2Ba, in an alternative configuration of the first preferred embodiment, the reservoir or blowing apparatus 3 uses a blower fan or turbine 21, which produces pressurized airflow after the concentrated oxygen flows through the first valve 14a. The blower fan 21 is positioned within a blower housing 3b of the reservoir 3. A preferred example of the blower fan 21 may be comprised of several different types of turbines used in continuous positive airway pressure ("CPAP") and bilevel positive airway pressure ("BiPAP") machines. For example, one such turbine is the RV centrifugal fan 21 from ebm-papst Inc. The RV centrifugal fan 21 can move air up to four hundred twenty liters per minute (420 L/min) at pressures of twenty (20) inches H₂O. The RV centrifugal fan 21 weighs approximately one hundred thirty-five grams (135g) and utilizes approximately forty-three Watts (43W) to operate. Another example of the turbine or blower fan 21 is an U65MN-KD-5 blower from Micronel AG. The turbine or blower fan 21 is contained in the blowing apparatus 3 which is attached to the blower housing 3b of the reservoir or blowing apparatus 3. The blowing apparatus 3 can also contain electronics and the rechargeable batteries 25. The preferred remote flow controller is attached to the blowing apparatus 3 that is fluidly connected to the oxygen concentrator 1 through the concentrated oxygen delivery tube 10 and the patient interface 5 through the patient delivery tube 10b. The oxygen concentrator preferably includes an oxygen controller device 9a for electronically controlling the pressure swing adsorption process. Alternatively, the reservoir or blowing apparatus 3 and the blower housing 3b may be incorporated together with the oxygen concentrator 1 and the associated housing 50 containing the batteries 25, the blower fan 21, associated electronics such as a controller for the blower fan 21 and various valves 14, 14a, 14b, 14c, 15, the sieve module 1a and the compressor 60 (See Fig. 2C). The remote flow controller can be permanently integrated into the reservoir 3, as shown in Fig. 5 at the blower controller device 9b or releasably secured thereto.

Referring to Fig. 2Bb, the second preferred embodiment includes the oxygen provided to the blowing apparatus 3 from the oxygen cylinder 29 as opposed to being provided from the portable oxygen concentrator 1. The flow of oxygen through the oxygen supply hose 10 is preferably regulated by a pressure regulator 11 attached to the cylinder 29.

Referring to Figs. 2A-2Ba and 4, the remote flow control or blowing apparatus 3 is preferably remotely positioned from the oxygen concentrator 1 and in relatively close proximity to the patient's nasal nares of the patient interface 5 so cannula pressure losses are minimized. An example of one potential system layout is shown in Fig. 4. The oxygen concentrator 1 is shown secured with a shoulder strap 30 to the patient for relatively convenient carrying by the patient but could also be contained in a backpack or similar carrying device. In an embodiment where the remote flow control or blowing apparatus 3 is adjacent, attached or removably attached to a backpack, it would provide a relatively close distance to the patient's nares from the remote flow control 3, as opposed to at the patient's waist location.

To maximize performance and minimize the size and energy use of the turbine or blower fan 21 in the reservoir or blowing apparatus 3, it is desirable to locate the blower fan 21 generally proximate the patient interface 5. Referring to Figs. 2A-2Ba, 4 and 11, a pressure differential ΔP for various tubing lengths and diameters is represented. The blower fan 21 produces an outlet gas flow for flow through a cannula connector 10d that diminishes as pressure restrictions such as delivery length, delivery curvature and interior tube obstructions are introduced, so the preferred portable ventilator 100 is sized to maintain the required pressure and flow at the patient interface 5.

The effects of mechanical ventilation with positive pressure on the venous return may be beneficial when used in patients with cardiogenic pulmonary edema. In these patients with volume overload, decreasing venous return will directly decrease the amount of pulmonary edema being generated, by decreasing right cardiac output. At the same time, the decreased return may improve overdistension in the left ventricle, placing it at a more advantageous point in the Frank-Starling curve and possibly improving cardiac output.

Proper management of mechanical ventilation also involves an understanding of lung pressures and lung compliance. Normal lung compliance is around one hundred (100) ml/cmH₂0. This means that in a normal lung the administration of five hundred milliliters (500 ml) of air via positive pressure ventilation will increase the alveolar pressure by five centimeters per water column (5 cm/H₂O). Conversely, the administration of a positive pressure of five centimeters per water column (5 cm/H₂O) will generate an increase in lung volume of five hundred milliliters (500 mL). Because ventilators are not always used on normal lungs, compliance may be much higher or much lower. Any disease that destroys lung parenchyma like emphysema will typically increase compliance and any disease that generates stiffer lungs (ARDS, pneumonia, pulmonary edema, pulmonary fibrosis) will typically decrease lung compliance.

The problem with stiff lungs is that small increases in volume can generate large increases in pressure and cause barotrauma. This generates a problem in patients with hypercapnia or acidosis as there may be a need to increase minute ventilation to correct these problems. Increasing respiratory rate may manage the increase in minute ventilation, but if increased respiratory rate is not feasible, increasing the tidal volume can increase plateau pressures and create barotrauma.

There are two main pressures in the system to be aware of when mechanically ventilating a patient: (a) peak pressure is the pressure achieved during inspiration when the air is being pushed into the lungs and is a measure of airway resistance; and (b) plateau pressure is the static pressure achieved at the end of a full inspiration. To measure plateau pressure, an inspiratory hold on the portable ventilator 100 is formed to permit for the pressure to equalize through the system. Plateau pressure is a measure of alveolar pressure and lung compliance. Normal plateau pressure is below thirty centimeters per water column (30 cm/H₂0), and higher pressure can generate barotrauma.

Providing ten to twenty centimeters per water column (10-20 cm/H₂O) is normally sufficient for ventilators delivering a tidal volume between ten and fifteen milliliters per kilogram (10-15mL/kg). Most modern ventilators can deliver flow rates between sixty and one hundred twenty liters per minute (60-120 L/min) with a peak flow rate of approximately sixty liters per minute (60 L/min) being adequate for most patients.

The oxygen concentrator 1 output is delivered to an inlet 3a of the blowing apparatus 3 and the blower fan 21 via the concentrated oxygen delivery tube 10. The length, size and composition of the concentrated oxygen delivery tube 10 generally determines how much oxygen flow is decreased to the blowing apparatus 3. The oxygen flow from the oxygen concentrator 1 through the concentrated oxygen delivery tube 10 adds additional energy to the blower fan 21 via a turbine inlet venturi 23, which raises the turbine inlet pressure. A blower motor 21a is mounted inside the blower housing 3b to drive and control the blower fan 21 during operation under direction of the blower controller device 9b. When a patient begins to inhale at the patient interface 5, the vacuum is sensed by a pressure sensor 5a, which can be incorporated into the patient interface 5 or located upstream of the cannula connector 10d along the gas pathway but downstream of the blower fan 21. The pressure sensor 5a preferably sends a signal to the blower controller device 9b, which sends a signal to the blower fan 21 to generate a predetermined volume of air that is delivered to the patient interface 5. At the same time the concentrated oxygen delivery tube 10 in the blower inlet venturi senses a negative pressure, the blower controller device 9b sends a signal to the oxygen controller device 9a and activates the oxygen concentrator 1 to deliver a bolus of O₂ as a result of the concentrator pressure sensor 5a sensing an inspiration event at the patient interface 5. The oxygen concentrator 1 may be adjacent to the ventilator, the blowing apparatus 3, or fluidly connected with a tube of up to fifty feet (50 ft) in length. The turbine or blower fan 21 can also be programmed such that PEEP can be maintained in the patient's lungs after each exhalation.

The blower controller device 9b is also preferably configured to increase or decrease the speed of the blower fan 21 based on the sensed pressure at the pressure sensor 5a. The blower controller device 9b is preferably able to control the speed of the blower fan 21 to match the breath pressure requirements of the patient and is able to change and adapt the speed of the blower fan 21 as the patient's breath pressure requirements change and shift with activity or for other reasons. The blower controller device 9b may be designed and configured to speed up and slow down the blower fan 21 so the pressure at the patient interface 5 generally follows the patient's breathing patterns and may be able to replace the valves 14a, 14b, 14c by controlling the speed fo the blower fan 21.

The outlet flow of the blower fan 21 can be controlled with the piezo valves 14, turbine motor speed control, or a combination of both that are directed by the blower controller device 9b. As with the first embodiment, different oxygen concentrator capacities can be used to achieve increased O₂ levels. Referring to Fig. 2Ba the oxygen concentrator 1 and remote flow control or blowing apparatus 3 arrangement of components includes the valve 15, the inlet filter 27, the turbine inlet venturi 23 and the cannula connector 10d. The Piezo valve 15 is shown controlling oxygen flow to the turbine inlet venturi 23 and the blower fan 21. Solenoid devices are the standard for electrically controlled pneumatic valves, however, the piezo valves 15 offer advantages over their solenoid counterparts and open entirely new areas of application. Pneumatic valves made with piezo technology, such as the piezo valves 15, offer many advantages. The piezo valves 14, 15 are small, lightweight, extremely precise, durable, fast, and save energy. The piezo valves 14, 15 do not need energy to maintain a switching status and, therefore, generate almost no heat. What's more, the piezo valves 14, 15 can potentially be operated without any or limited noise. Another advantage is that the piezo valves 14, 15 work proportionally.

The piezo valves 14, 15 can be a better alternative to conventional solenoid valves, especially in applications requiring directly controlled proportional valves. The piezo valves 14, 15 provide relatively gentle and safe speed control for pneumatic cylinders, and work well in medical applications, laboratory automation, manufacturing, and even motor vehicles.

Referring to Fig. 2C, in a third preferred embodiment, the oxygen concentrator 1 includes the remote flow control or blowing apparatus 3 and blower fan 21 along with all the valves and electronics contained within the same housing 50, thereby incorporating the blowing apparatus 3 and the blower housing 3b together with the housing 50. The preferred components include, but are not limited to, the blower controller device 9b, the valves 14 or the piezo valves 15, the batteries 25 and the blower fan 21. This configuration provides a compact design in terms of patient use and portability. The oxygen concentrator 1 includes a removably mounted cartridge or sieve module 1a located within the housing 50 that is in fluid connection with a compressor 60 that is mounted inside the housing 50. The cartridge or sieve module 1a contains zeolite for removing nitrogen from air through a pressure swing adsorption process for creating concentrated oxygen. The batteries 25 or an external power source may also be attached to or contained within the housing 50. The oxygen controller or controller device 9a is also attached to the housing 50 for electronically controlling the pressure swing adsorption process of the oxygen concentrator 1. The oxygen controller device 9a preferably electronically controls the pressure swing adsorption process of the portable oxygen concentrator 1.

This third preferred system 1x does, however, reduce the modularity described in the first and second preferred embodiments of Figs. 2B, 2Ba and 2Bb, wherein various concentrators or oxygen cylinders can be releasably attached to the remote flow control and the reservoir 3. The third preferred system 1x does expect that the delivery tubing or patient delivery tube 10b be kept as short as possible or at least reasonably short (see Fig. 2C). Once again, to prevent parasitic pressure differential ΔP losses resulting from tubing friction, a backpack design as described herein would allow for a shorter air and oxygen delivery cannula or patient delivery tube 10b to the patient interface 5, although such a configuration is not required or limiting. This design is most applicable when the system 1x is carried by the shoulder strap 30 or placed close to the patient. In an alternative embodiment (not shown), the flow control, reservoir or blowing apparatus 3 is preferably, removably attached to the oxygen concentrator 1. This configuration allows the oxygen concentration 1 and components to be compressed into a single construct while giving the patient the opportunity to disengage the components if it is more convenient. Such a modular configuration allows a different oxygen concentrator 1 with different capabilities to be removably attached to the remote flow control or blowing apparatus 3 providing the patient with the opportunity to change oxygen and air sources. An anticipated weight of this preferred system is four to five pounds (4-5 lbs.) based on existing oxygen concentrator product parameters. An oxygen outlet port is preferably provided for bottled oxygen for emergency backup (see Fig. 2C).

Referring to Fig. 2B, the patient interface 5, which is preferably comprised of a mask 5, transmits a pressure drop to the remote flow control or blowing apparatus 3 indicating the beginning of inspiration. Software preferably causes the initiation of the blower fan or turbine 21 rotations per minute ("RPM ") to ramp and increase flow through to the blower fan 21 and increase pressure delivery to the patient. This sequence of events causes a reduction in pressure at the blower inlet or inlet filter 27. This pressure reduction is transmitted to the concentrator 1. The concentrator 1 is preferably in pulse dose mode, so a bolus of oxygen is delivered through to concentrated oxygen delivery tube 10 to the blower venturi inlet or inlet filter 27. The bolus volume is preferably determined by the concentrator level setting and a breath per minute ("BPM") / bolus quotient look-up table which uses a moving average of breaths per minute. The bolus delivery at the venturi throat increases the inlet pressure of the blower 21, thereby causing an increase in blower efficiency. The oxygen is delivered with the ventilation air and at the same time that inspiration is detected. Any size oxygen concentrator 1 may be used [in conjunction with the device that is the subject of the present disclosure], depending on the physician's recommendation. An optional piezo valve 14, 15 may control the flow of oxygen to the turbine inlet venturi 23 through the blower controller 9b.

In the event of a power failure, the cylinder 29 of pressurized oxygen of the second preferred embodiment may be used in place of the concentrator 1 and oxygen pressure may be used to cause air to be entrained into the venturi 23 and then flow through the blower 21 to the patient interface or mask 5. Given sufficient pressure from the oxygen cylinder 29, sufficient flow and positive pressure can be developed to provide the patient with positive pressure air.

When the battery 25 becomes discharged to the point that it can no longer support the operation of the blower 21, an alarm is delivered and the blower 21 is shut off. Limited, but sufficient battery power then remains to operate the valves 14, 15 so that inspiration causes its operation and air/oxygen delivery is sustained for several hours.

Referring to Fig. 5, top and side views of the integrated remote flow control or blowing apparatus 3 with the blower controller device 9b is contained within the blower housing 3b.

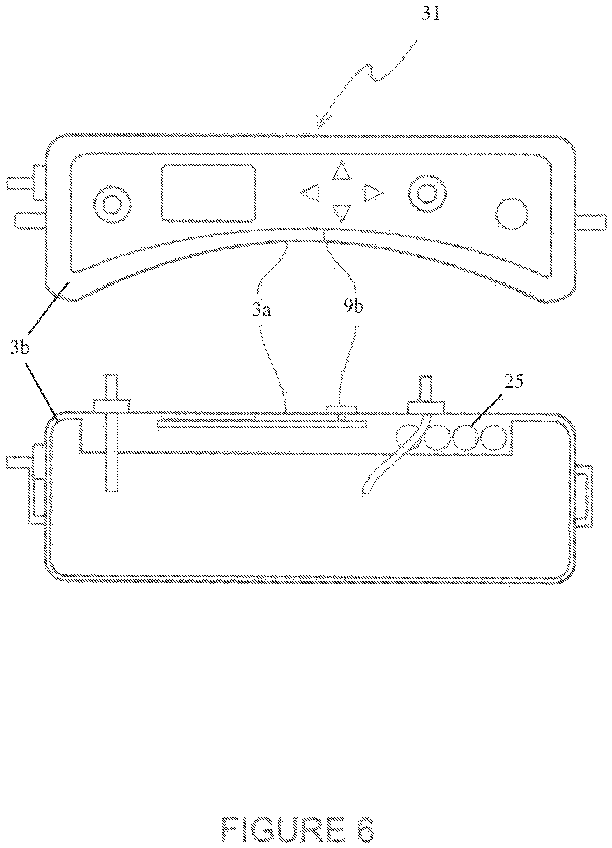

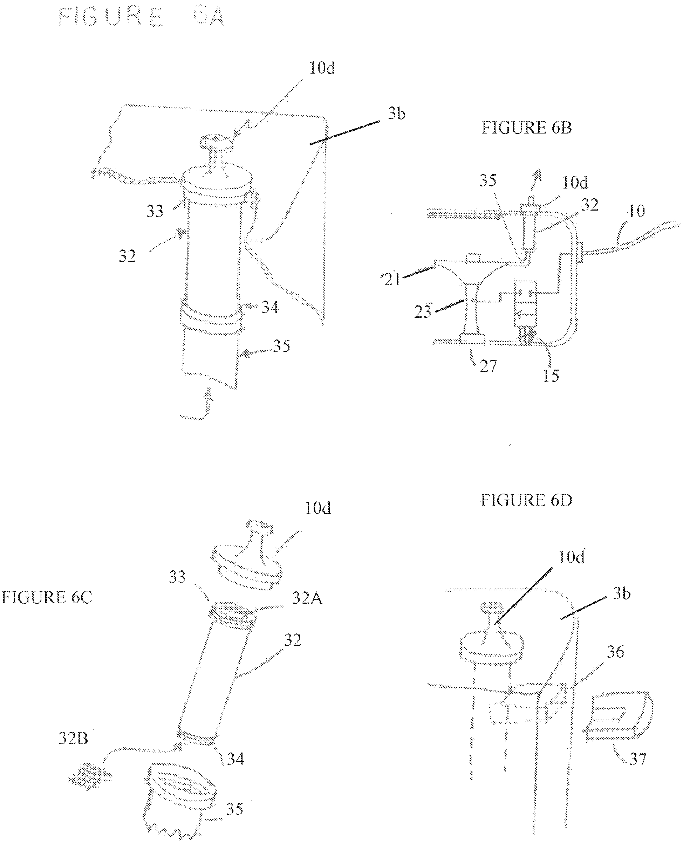

Referring to Fig. 6, the integrated flow control or blowing apparatus 3 collectively comprise a pressure control 31.

Referring to Figs. 6A-6D, an alternative embodiment of the flow control or blowing apparatus 3 includes a medicant delivery apparatus 32 deployed downstream from the blower fan 21. In one preferred embodiment, the medicant delivery apparatus 32 is a removable hollow cartridge with a threaded distal end 33 and a threaded proximal end 34. The interior of delivery apparatus 32 can be lined with a drug or moisture eluting polymer 32A such as a polyether block amide, commercially known as PEBAX, that is spiked or impregnated with a medication that elutes as gas from the blower fan 21 flows through the medicant delivery apparatus 32. In another preferred embodiment, a polymer mesh 32B is located within the medicant delivery apparatus 32. As gas flows from the blower fan 21 through the medicant delivery apparatus 32 and through the mesh 32b, the mesh 32B and moisture eluting polymer 32A wick medication for delivery to a patient through the nasal cannula and the patient interface 5. The medication is then inhaled with each inspiration by the patient. In one preferred embodiment, the cannula connector 10d has threading to releasably connect to the medicant delivery apparatus 32 and threading to releasably connect to the housing 50 or the blower housing 3b of the blowing apparatus 3. The threaded proximal end 34 threadably connects to a gas output 35 that is fluidly connected to the blower fan 21.

Alternative preferred embodiments of the medicant delivery apparatus 32 include a small electronic atomizer, nebulizer or vaporizer 37 that generates a medical aerosol simultaneously with the flow of gas from the blower fan 21 to be delivered to the patient. Depending on the required medications of the user, different medications may be provided in medicant delivery apparatus 32. In one preferred embodiment, the atomizer 37 is releasably received in a receiving port 36. The receiving port 36 is electrically connected to the batteries 25 and the blower controller device 9b. The receiving port 36 is connected to the medicant delivery apparatus 32 through a one way valve that allows medication from the atomizer 32 to be delivered into the delivery apparatus 32 as determined by the blower controller device 9b. Alternatively, the delivery apparatus 32 or the atomizer 37 may be quick connected to the cannula connector 10d or may be integrated into the patient delivery tube 10b. In this alternative preferred embodiment, the delivery apparatus 32 or atomizer 37 is preferably mounted in the concentrated oxygen stream outside of the housing 50 and blower housing 3b such that the delivery apparatus or atomizer 37 may be quickly and easily removed and replaced such that the user is able to quickly add or remove medication or other therapeutics depending on patient needs or prescriptions.

In another preferred embodiment, an ozone generator (not shown) is located adjacent the venturi 23. Ozone is an oxidizer that has been shown to kill bacteria and mold. An ozone generator will produce ozone by adding energy to oxygen molecules (O₂), which cause the oxygen atoms to part ways and temporarily recombine with other O₂ molecules creating ozone (O₃). The ozone generator creates ozone by introducing concentrated oxygen from the concentrator 1 into a high voltage chamber that creates ozone through a process known as Corona Discharge. In one preferred embodiment, the blower fan 21 and tubing from the blower fan 21 through to the patient interface 5 is sanitized by ozone by generating ozone at the ozone generator and then blowing the ozone through the blower fan 21 and tubing to the interface 5. Recognizing the dangers of a patient inhaling large amounts of ozone, the system 1 preferably includes a safety mechanism preventing use of the interface while ozone cleaning is occurring. For example, the interface 5 would have to be secured to a tray or receiving cartridge in order for ozone generation to occur. The ozone generator is preferably located adjacent to the venturi 23 or alternatively as a component of the oxygen concentrator 1 (see US Patent No. 9,492,781, see also, for example, Fig 17 and claim 17).

In another preferred embodiment, a pulse oximeter (not shown) is connected to a patient's finger, neck, wrist, ear lobe or other acceptable location for measuring blood oxygen saturation levels. The pulse oximeter is preferably, wirelessly connected to the oxygen controller device 9a or the blower controller device 9b. The preferred pulse oximeter could be a traditional finger secured unit such as the MightySat from Masimo or the AppleWatch from Apple. Such wireless connection between a pulse oximeter and the oxygen controller device 9a or the blower controller device 9b can be a passive connection where information received from the pulse oximeter is used by a caregiver or the patient to adjust a patient's air and oxygen flow or the connection can be an active connection where the pulse oximeter information or collected data is used to automatically adjust the patient's oxygen and air flow settings to achieve a desired blood oxygen saturation level. In a further preferred embodiment, the pulse oximeter is connected to the oxygen or blower controller device 9a, 9b and a remote computer such as a laptop or phone which contains additional memory storage options, settings, controls, web-based information such as temperature, location, speed, altitude, humidity and pressure. Such additional factors can be used to further adjust a patient's oxygen and air flow while also creating a history of such information for a caregiver to review. It is further contemplated, for example, that such historical data related to the patient's oxygen saturation levels, could be used to pre-empt blood oxygen saturation levels from dropping if historical data shows a pattern that can be anticipated. In one preferred embodiment, a cell phone with a global positioning system ("GPS") tracker creates a historical map of a travel path of the patient. In one such situation, the patient has historically shown a drop of blood oxygen saturation level upon walking up an incline known on the map. To pre-empt the patient from becoming desaturated below a preferred level, the phone sends a command to the oxygen or blower controller device 9a, 9b to increase oxygen and air flow in anticipation of the incline.

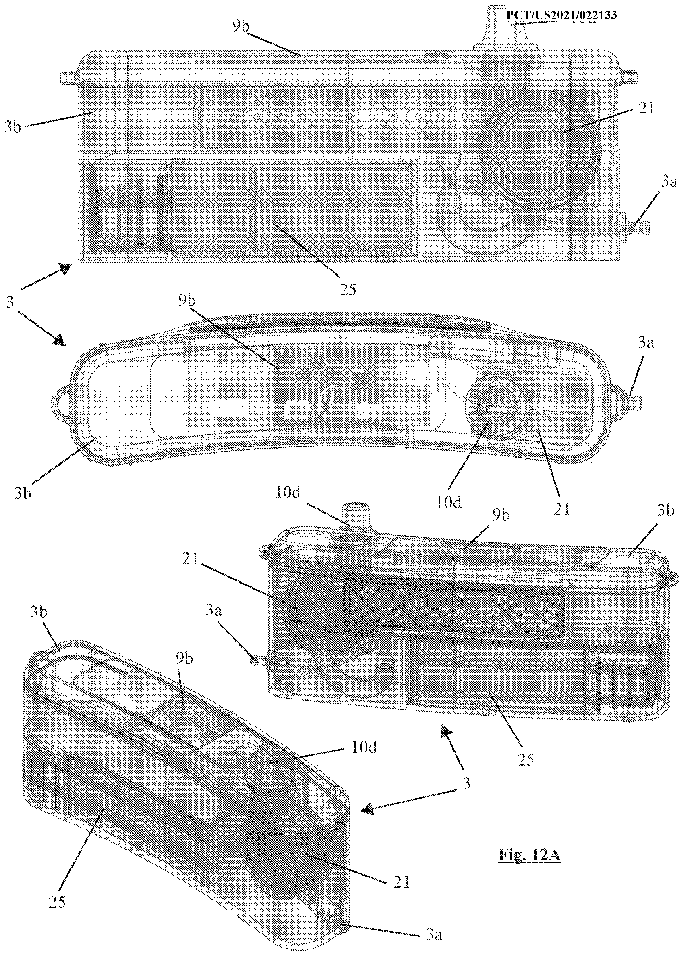

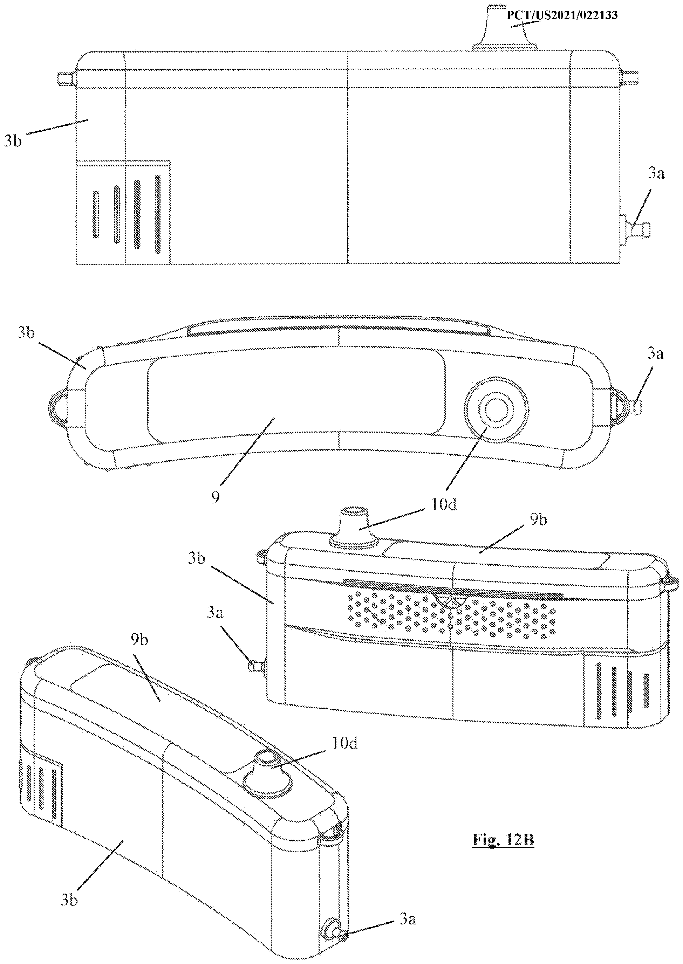

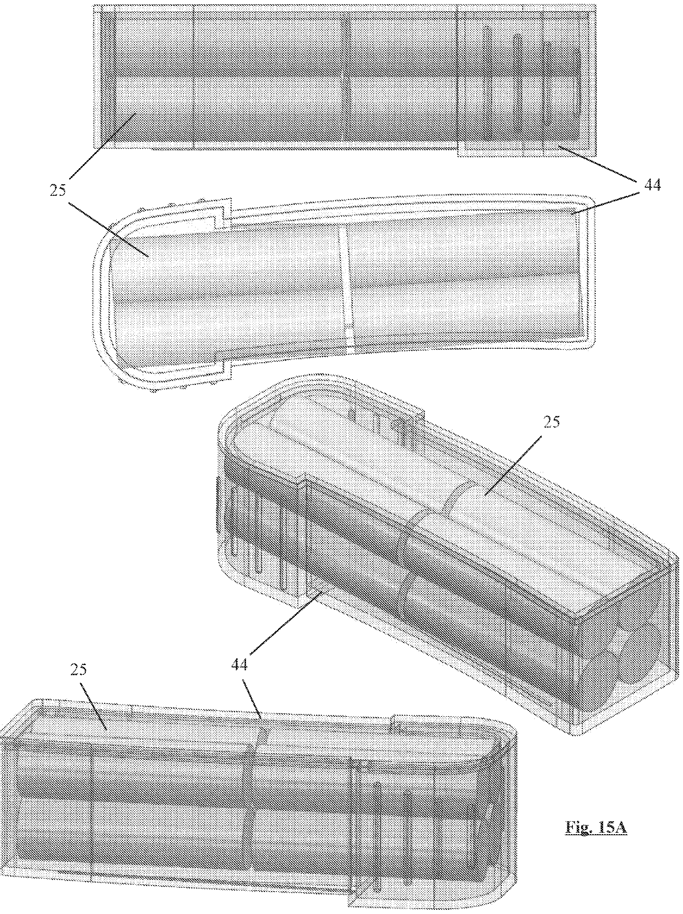

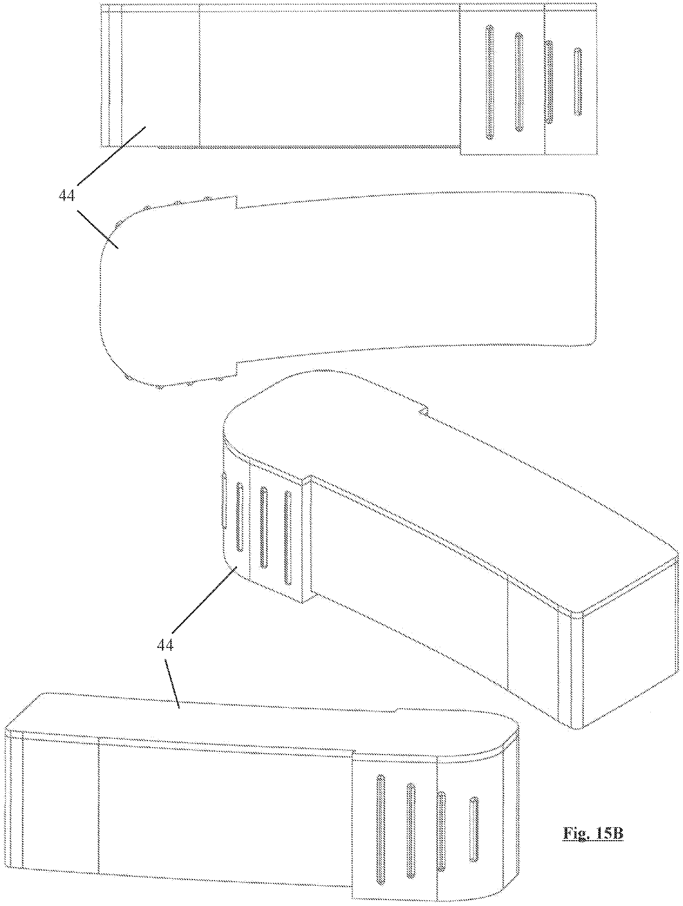

Referring to Figs. 12A-15B, a preferred embodiment of a portable ventilator 1 includes the components of the first, second and third preferred embodiments described above in the housings and modules shown in Figs. 12A-15B.

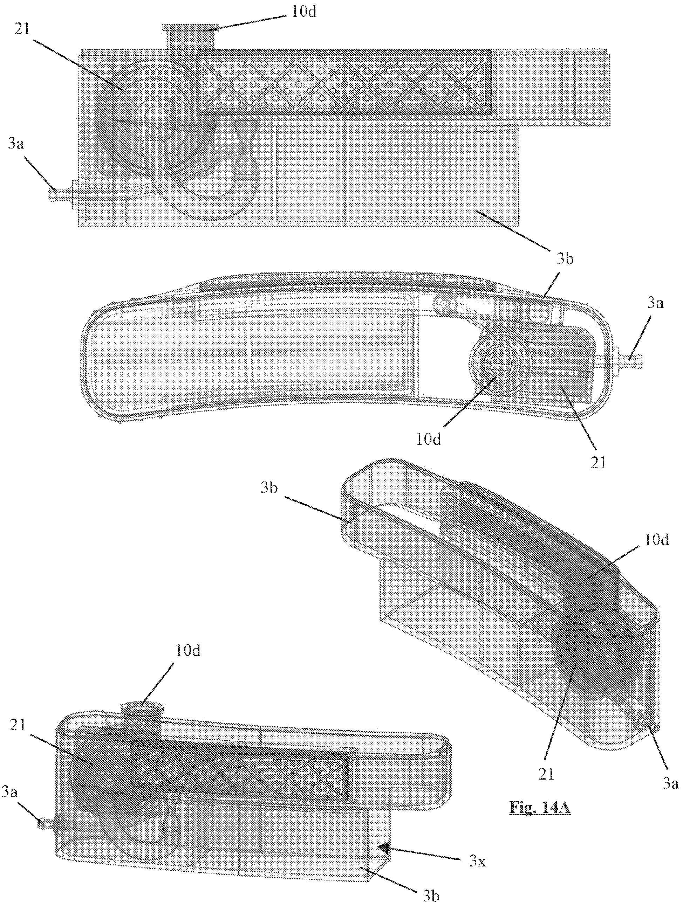

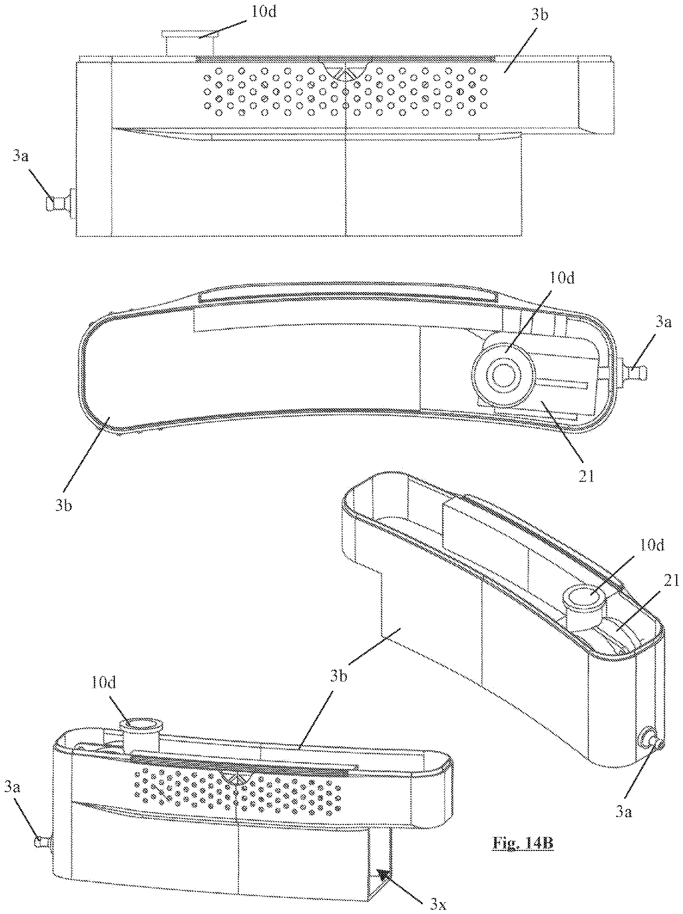

Referring to Figs. 14A-15B, the blowing apparatus 3 may include a battery housing 44 that is removably mountable in a battery cavity 3x in the blower housing 3b. The battery housing 44 is preferably slidably mountable in the battery cavity 3x such that the rechargeable batteries 25 may be removed and recharged or replaced during operation. The blowing apparatus 3 is not limited to including the battery housing 44 that is removably mountable in the battery cavity 3x or to including the rechargeable batteries 25 and may be otherwise powered and the blowing apparatus 3 may be otherwise powered during operation.

The scope of the present invention is defined by the following appended claims.

## Claims

1. An oxygen delivery system (100) for delivering concentrated oxygen to a patient, the oxygen delivery system (100) comprising:
an oxygen concentrator (1) having a housing (50);
a compressor (60) mounted inside the housing (50);
a sieve module (1a) located within the housing (50) and in fluid connection with the compressor (60), the sieve module (1a) containing a zeolite for removing nitrogen from air through a pressure swing adsorption process for creating concentrated oxygen;
a power source (61) attached to the housing (50); an oxygen controller device (9a) for electronically controlling the pressure swing adsorption process; and
a blowing apparatus (3) in fluid communication with the oxygen concentrator (1), wherein the compressor is in fluid communication with the blowing apparatus (3) through a concentrated oxygen delivery tube (10);
the blowing apparatus (3) comprising:
a blower housing (3b);
a blower motor (21a) mounted inside the blower housing (3b);
a blower fan or turbine (21) connected to the blower motor (21a);
a turbine inlet venturi (23);
a second power source (25) attached to the blower housing (3b); and
a blower controller device (9b) for electronically controlling the blowing apparatus (3);
and wherein the oxygen flow from the oxygen concentrator (1) through the concentrated oxygen delivery tube (10) adds additional energy to the blower fan or turbine (21) via the turbine inlet venturi (23), which raises the turbine inlet pressure.

2. The oxygen delivery system (100) of claim 1, further comprising: a medicant delivery apparatus (32) mounted to the blower housing (3b) and deployed downstream from the blower fan or turbine (21); wherein an interior of the medicant delivery apparatus (32) is lined with a drug or moisture eluting polymer (32A).

3. The oxygen delivery system (100) of claim 2, wherein the drug or moisture eluting polymer (32A) is comprised of a polyether block amide.

4. The oxygen delivery system (100) of claim 2, wherein the drug or moisture eluting polymer (32A) is impregnated with a medication that elutes as gas from the blower fan or turbine (21) flows through the medicant delivery apparatus (32).

5. The oxygen delivery system (100) of claim 1, wherein ambient air is introduced to the blower fan or turbine (21) through an inlet filter (27) attached to the blower housing (3b).

6. The oxygen delivery system (100) of claim 1 further comprising:
a patient interface (5) in fluid communication with the blowing apparatus, the patient interface (5) configured to provide concentrated oxygen to the patient.

7. The oxygen delivery system (100) of claim 1, further comprising:
a valve (14, 14a, 14b, 14c, 15) positioned within the blower housing (3b), the valve (14, 14a, 14b, 14c, 15) controlling flow of concentrated oxygen from the compressor (60) to the blower fan or turbine (21).

8. The oxygen delivery system (100) of claim 1, wherein the sieve module (1a) is removably mountable within the housing (50).

9. The oxygen delivery system (100) of claim 1, wherein the power source (61) is comprised of a rechargeable battery removably mounted to the housing (50).

10. The oxygen delivery system (100) of claim 1, wherein the second power source (25) is comprised of a rechargeable battery removably mounted to the blower housing (3b).

11. The oxygen delivery system (100) of claim 1, wherein the blower controller device (9b) is configured to increase or decrease the speed of the blower fan or turbine (21) to control flow of ambient air and oxygen through the blower housing (3b) and to the patient.

12. The oxygen deliver system (100) of claim 1, wherein the blowing apparatus (3) is in fluid communication with the compressor (60) through the sieve module (1a) and the concentrated oxygen delivery tube (10) extending between the housing (50) and the blower housing (3b).

13. The oxygen delivery system (100) of claim 1, wherein the blowing apparatus (3) is controlled via a wireless connection.

14. The oxygen delivery system (100) of claim 1, wherein the oxygen controller device (9a) or the blower controller device (9b) is wirelessly connected to a pulse oximeter for measuring blood oxygen saturation levels of the patient

15. The oxygen delivery system (100) of claim 14, wherein data collected from the pulse oximeter is used to automatically adjust an oxygen and air flow setting of the patient to achieve a desired blood oxygen saturation level.

## Patentansprüche

1. Sauerstoffabgabesystem (100) zum Abgeben von konzentriertem Sauerstoff an einen Patienten, wobei das Sauerstoffabgabesystem (100) aufweist:
einen Sauerstoffkonzentrator (1) mit einem Gehäuse (50);
einen Kompressor (60), der innerhalb des Gehäuses (50) angebracht ist;
ein Siebmodul (la), das sich innerhalb des Gehäuses (50) befindet und in Fluidverbindung mit dem Kompressor (60) steht, wobei das Siebmodul (la) ein Zeolith zum Entfernen von Stickstoff aus Luft durch einen Druckwechseladsorptionsprozess zum Erzeugen von konzentriertem Sauerstoff enthält;
eine Leistungsquelle (61), die an dem Gehäuse (50) angebracht ist;
eine Sauerstoffcontrollereinrichtung (9a) zum elektronischen Steuern des Druckwechseladsorptionsprozesses; und
eine Gebläsevorrichtung (3) in Fluidkommunikation mit dem Sauerstoffkonzentrator (1),
wobei der Kompressor durch eine Röhre (10) zur Abgabe von konzentriertem Sauerstoff mit der Gebläsevorrichtung (3) in Fluidkommunikation steht;
wobei die Gebläsevorrichtung (3) aufweist:
ein Gebläsegehäuse (3b);
einen Gebläsemotor (21a), der im Inneren des Gebläsegehäuses (3b) montiert ist;
einen Gebläselüfter oder eine Gebläseturbine (21), die mit dem Gebläsemotor (21a) verbunden ist;
einen Venturi-Turbineneinlass (23);
eine zweite Leistungsquelle (25), die an dem Gebläsegehäuse (3b) angebracht ist; und
eine Gebläsecontrollereinrichtung (9b) zum elektronischen Steuern der Gebläsevorrichtung (3);
und wobei der Sauerstoffstrom von dem Sauerstoffkonzentrator (1) durch die Röhre (10) zur Abgabe von konzentriertem Sauerstoff dem Gebläselüfter oder der Gebläseturbine (21) über den Venturi-Turbineneinlass (23) zusätzliche Energie zuführt, wodurch der Turbineneinlassdruck erhöht wird.

2. Sauerstoffabgabesystem (100) nach Anspruch 1, das weiterhin aufweist:
eine Medikamentenabgabevorrichtung (32), die an dem Gebläsegehäuse (3b) montiert ist und stromabwärts von dem Gebläselüfter oder der Gebläseturbine (21) stationiert ist; wobei ein Inneres der Medikamentenabgabevorrichtung (32) mit einem Arzneimittel oder Feuchtigkeit eluierenden Polymer (32A) ausgekleidet ist.

3. Sauerstoffabgabesystem (100) nach Anspruch 2, wobei das Arzneimittel oder Feuchtigkeit eluierende Polymer (32A) aus einem Polyetherblockamid besteht.

4. Sauerstoffabgabesystem (100) nach Anspruch 2, wobei das Arzneimittel oder Feuchtigkeit eluierende Polymer (32A) mit einem Medikament imprägniert ist, das eluiert, wenn Gas von dem Gebläselüfter oder der Gebläseturbine (21) durch die Medikamentenabgabevorrichtung (32) strömt.

5. Sauerstoffabgabesystem (100) nach Anspruch 1, wobei Umgebungsluft durch einen an dem Gebläsegehäuse (3b) angebrachten Einlassfilter (27) in den Gebläselüfter oder die Gebläseturbine (21) eingeleitet wird.

6. Sauerstoffabgabesystem (100) nach Anspruch 1, das weiterhin aufweist:
eine Patientenschnittstelle (5), die mit der Gebläsevorrichtung in Fluidverbindung steht, wobei die Patientenschnittstelle (5) dazu ausgebildet ist, dem Patienten konzentrierten Sauerstoff zuzuführen.

7. Sauerstoffabgabesystem (100) nach Anspruch 1, das weiterhin aufweist:
ein Ventil (14, 14a, 14b, 14c, 15), das in dem Gebläsegehäuse (3b) positioniert ist, wobei das Ventil (14, 14a, 14b, 14c, 15) den Fluss von konzentriertem Sauerstoff von dem Kompressor (60) zu dem Gebläselüfter oder zu der Gebläseturbine (21) steuert.

8. Sauerstoffabgabesystem (100) nach Anspruch 1, wobei das Siebmodul (la) entfernbar in dem Gehäuse (50) montierbar ist.

9. Sauerstoffabgabesystem (100) nach Anspruch 1, wobei die Leistungsquelle (61) eine wiederaufladbare Batterie, die entfernbar an dem Gehäuse (50) montiert ist, aufweist.

10. Sauerstoffabgabesystem (100) nach Anspruch 1, wobei die zweite Leistungsquelle (25) eine wiederaufladbare Batterie, die entfernbar an dem Gebläsegehäuse (3b) montiert ist, aufweist.

11. Sauerstoffabgabesystem (100) nach Anspruch 1, wobei die Gebläsecontrollereinrichtung (9b) dazu ausgebildet ist, die Geschwindigkeit des Gebläselüfters oder der Gebläseturbine (21) zu erhöhen oder zu verringern, um den Fluss von Umgebungsluft und Sauerstoff durch das Gebläsegehäuse (3b) und zu dem Patienten zu steuern.

12. Sauerstoffabgabesystem (100) nach Anspruch 1, bei dem die Gebläsevorrichtung (3) durch das Siebmodul (la) und die Röhre (10) für die Abgabe von konzentriertem Sauerstoff, die sich zwischen dem Gehäuse (50) und dem Gebläsegehäuse (3b) erstreckt, in Fluidverbindung mit dem Kompressor (60) steht.

13. Sauerstoffabgabesystem (100) nach Anspruch 1, wobei die Gebläsevorrichtung (3) über eine drahtlose Verbindung gesteuert wird.

14. Sauerstoffabgabesystem (100) nach Anspruch 1, wobei die Sauerstoffcontrollereinrichtung (9a) oder die Gebläsecontrollereinrichtung (9b) drahtlos mit einem Pulsoximeter zum Messen der Blutsauerstoffsättigungspegel des Patienten verbunden ist.

15. Sauerstoffabgabesystem (100) nach Anspruch 14, wobei von dem Pulsoximeter gesammelte Daten verwendet werden, um eine Sauerstoff- und Luftstromeinstellung des Patienten automatisch einzustellen, um einen gewünschten Blutsauerstoffsättigungspegel zu erreichen.

## Revendications

1. Système d'administration d'oxygène (100) pour distribuer de l'oxygène concentré impatient,
le système d'administration d'oxygène (100) comprenant :
- un concentrateur d'oxygène (1) ayant un boîtier (50),
- un compresseur (60) logé dans le boîtier (50),
- un module de tamis (1a) dans le boîtier (50) et en liaison fluidique avec le compresseur (60), le module de tamis (1a) comportant de la zéolite pour enlever l'azote de l'air par un procédé d'adsorption par variation de pression pour obtenir l'oxygène concentré,
- une source d'énergie (61) fixée au boîtier (50),
- un dispositif de contrôle d'oxygène (9a) pour contrôler électroniquement le procédé d'adsorption par variation de pression ; et une soufflante (3) en liaison fluidique avec le concentrateur d'oxygène (1),
système dans lequel
le compresseur est en liaison fluidique avec la soufflante (3) par un tube de distribution d'oxygène concentré (10),
- la soufflante (3) comprenant :
* un boîtier de soufflante (3b),
* un moteur de soufflante (21a) à l'intérieur du boîtier de soufflante (3b),
* un ventilateur ou turbine (21) relié au moteur de soufflante (21a),
* un venturi d'entrée de turbine (23),
- une seconde source d'énergie (25) fixée au boîtier de soufflante (3b), et
- un dispositif de commande de soufflante (9b) pour commander électroniquement la soufflante (3), et
- le flux d'oxygène du concentrateur d'oxygène (1) à travers le tube de distribution d'oxygène concentré (10) ajoutant de l'énergie supplémentaire à la turbine ou au ventilateur (21) par le venturi d'entrée de turbine (23) qui augmente la pression d'entrée de la turbine.

2. Système d'administration d'oxygène (100) selon la revendication 1, comprenant en outre :
- un appareil de distribution de médicaments (32) installé sur le boîtier de soufflante (3b) et déployé en aval du ventilateur ou turbine,
dans lequel
l'intérieur de l'appareil de distribution de médicament (32) est recouvert d'un polymère d'élution de médicaments ou d'humidité (32A).

3. Système d'administration d'oxygène (100) selon la revendication 2,
dans lequel
le polymère d'élution de médicaments ou d'humidité (32A) est un amide bloc polyéther.

4. Système d'administration d'oxygène (100) selon la revendication 2,
dans lequel
le polymère d'élution de médicaments ou d'humidité (32A) est imprégné d'un médicament qui s'élue comme gaz du ventilateur ou turbine (21) passant à travers l'appareil de distribution de médicament (32).

5. Système d'administration d'oxygène (100) selon la revendication 1,
dans lequel
l'air ambiant alimente le ventilateur ou turbine (21) en passant par un filtre d'entrée (27) fixé au boîtier de soufflante (3b).

6. Système d'administration d'oxygène (100) selon la revendication 1, comprenant en outre :
- une interface patient (5) en liaison fluidique avec la soufflante, l'interface de patient (5) étant configurée pour fournir de l'oxygène concentré au patient.

7. Système d'administration d'oxygène (100) selon la revendication 1, comprenant en outre :
- une soupape (14, 14a, 14b, 14c, 15) placée dans le boîtier de soufflante (3b), la soupape (14, 14a, 14b, 14c, 15) contrôlant le flux d'oxygène concentré du compresseur (60) vers le ventilateur ou turbine (21).

8. Système d'administration d'oxygène (100) selon la revendication 1,
dans lequel
le module de tamis (1a) est monté de manière amovible dans le boîtier (50).

9. Système d'administration d'oxygène (100) selon la revendication 1,
dans lequel
la source d'énergie (61) se compose d'une batterie rechargeable installée de manière amovible dans le boîtier (50).

10. Système d'administration d'oxygène (100) selon la revendication 1,
dans lequel
la seconde source d'énergie (25) est une batterie rechargeable installée de manière amovible sur le boîtier de soufflante (3b).

11. Système d'administration d'oxygène (100) selon la revendication 1,
dans lequel
le dispositif de contrôle de soufflante (9b) est configuré pour augmenter ou diminuer la vitesse du ventilateur ou de la turbine (21) pour commander le flux d'air ambiant et d'oxygène à travers le boîtier de soufflante (3b) et vers le patient.

12. Système d'administration d'oxygène (100) selon la revendication 1,
dans lequel
la soufflante (3) est en liaison fluidique avec le compresseur (60) à travers le module de tamis (1a) et le tube de distribution d'oxygène concentré (10) relie le boîtier (50) et le boîtier de soufflante (3b).

13. Système d'administration d'oxygène (100) selon la revendication 1,
dans lequel
la soufflante (3) est commandée par une liaison par fil.

14. Système d'administration d'oxygène (100) selon la revendication 1,
dans lequel
l'appareil de commande d'oxygène (9a) ou le dispositif de commande de soufflante (9b) est relié sans fil à un oxymètre à impulsions pour mesurer les niveaux de saturation d'oxygène sanguin du patient.

15. Système d'administration d'oxygène (100) selon la revendication 14,
dans lequel
les données collectées par l'oxymètre à impulsions sont utilisées pour régler automatiquement l'oxygène ou le flux d'oxygène et d'air du patient pour arriver au niveau de saturation voulu d'oxygène sanguin.
